# EUROPÄISCHE PATENTSCHRIFT

(11) **EP 0 658 551 B1**
(45) Veröffentlichungstag und Bekanntmachung des Hinweises auf die Patenterteilung: **19.05.1999**
(21) Anmeldenummer: 94119130.6
(22) Anmeldetag: 05.12.1994
(51) Int. Cl.: C07D 273/00, C07K 11/02, A61K 31/395

(54) **Neue cyclische Depsipeptide mit 18 Ringatomen und ihre Verwendung zur Bekämpfung von Endoparasiten**
Cyclic depsipeptides having 18 ringatoms and their use in controlling endoparasites
Depsipeptides cycliques ayant un cycle à 18 atomes et leur utilisation dans la lutte contre les endoparasites

(30) Priorität: 16.12.1993 DE 4342907
(43) Veröffentlichungstag der Anmeldung: 21.06.1995
(73) Patentinhaber: BAYER AG, 51368 Leverkusen (DE)
(72) Erfinder: Jeschke, Dr. Peter, D-51373 Leverkusen (DE); Scherkenbeck, Dr. Jürgen, D-42929 Wermelskirchen (DE); Bonse, Dr. Gerhard, D-51061 Köln (DE); Bischoff, Dr. Erwin, D-42115 Wuppertal (DE); Mencke, Dr. Norbert, D-51381 Leverkusen (DE); Harder, Dr. Dr. Achim, D-51109 Köln (DE); Londershausen, Dr. Michael, D-40699 Erkrath (DE); Müller, Dr. Hartwig, D-42553 Velbert (DE)

(56) Entgegenhaltungen:
- WO-A-93/25543
- CANADIAN JOURNAL OF CHEMISTRY., Bd.70, Nr.5, Mai 1992, OTTAWA CA Seiten 1281 - 1287 L. A. BLAIS ET AL 'Isolation and characterization of enniatins from fusarium avenaceum DAOM 196490'
- CHEMICAL ABSTRACTS, vol. 83, no. 13, 29. September 1975, Columbus, Ohio, US; abstract no. 114872e, V. Z. PLETNEV ET AL 'Theoritical conformational analysis of cyclic hexadepsipeptides with LLLLLL and LDLLDL residue configurations' Seite 606 ; & BIOORG. KHIM., Bd.1, Nr.2, 1975 Seiten 160 - 165

## Beschreibung

Die vorliegende Erfindung betrifft neue cyclische Depsipeptide mit 18 Ringatomen, ein Verfahren zu ihrer Herstellung und ihre Verwendung zur Bekämpfung von Endoparasiten.

Bestimmte cyclische Depsipeptide mit 18 Ringatomen (Enniatine) und Verfahren zu ihrer Herstellung und ihre Verwendung als Endoparasitizide sind Gegenstand einer vorgängigen, jedoch nicht vorveröffentlichten Patentanmeldung (Deutsche Patentanmeldung DE-OS 4 317 458.2).

Die Wirksamkeit dieser vorbekannten Verbindungen ist jedoch bei niedrigen Aufwandmengen und Konzentrationen nicht völlig zufriedenstellend.

Die vorliegende Erfindung betrifft:
1. Neue cyclische Depsipeptide mit 18 Ringatomen der allgemeinen Formel (I) 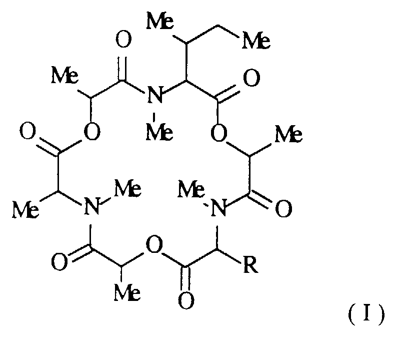 in welcher
   - R: für Wasserstoff, geradkettiges oder verzweigtes oder cyclisches Alkyl mit bis zu 8 Kohlenstoffatomen steht
   sowie deren optische Isomere und Racemate.
2. Verfahren zur Herstellung der neuen cyclischen Depsipeptide mit 18 Ringatomen der allgemeinen Formel (I) 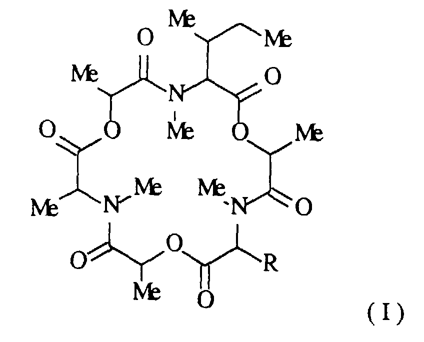 in welcher
   - R: für Wasserstoff, geradkettiges oder verzweigtes oder cyclisches Alkyl mit bis zu 8 Kohlenstoffatomen steht
   sowie deren optische Isomere und Racemate,
   dadurch gekennzeichnet, daß man
   offenkettige Hexadepsipeptide der allgemeinen Formel (II) 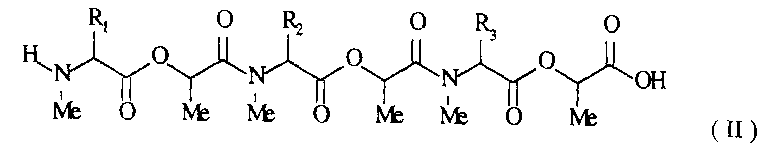 in welcher
   einer der Reste R¹,R² und R³ für Wasserstoff, geradkettiges oder verzweigtes oder cyclisches Alkyl mit bis zu 8 Kohlenstoffatomen steht und die anderen beiden Reste für Methyl und sec.-Butyl stehen,
   in Gegenwart eines geeigneten Kupplungsreagenzes, in Gegenwart eines basischen Reaktionshilfsmittels und in Gegenwart eines Verdünnungsmittels cyclisiert.

Die neuen cyclischen Hexadepsipeptide der allgemeinen Formel (I) sowie deren optische Isomere und Racemate sowie deren Säureadditions-Salze und Metallsalz-Komplexe eignen sich hervorragend zur Bekämpfung von Endoparasiten, besonders auf dem Gebiet der Medizin und Veterinärmedizin.

Die Herstellung der offenkettigen Hexadepsipeptide der Formel (II) ist in einer vorgängigen, jedoch nicht vorveröffentlichten Patentanmeldung beschrieben (vgl. Deutsche Patentanmeldung P 4 317 458.2)

Man erhält die Verbindungen der allgemeinen Formel (II) 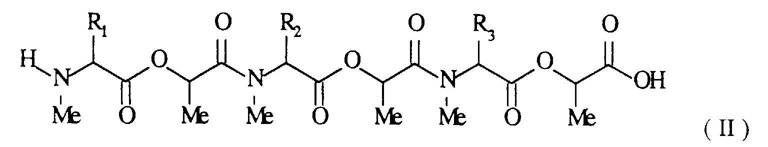 in welcher
R¹, R² und R³ die weiter oben angegebene Bedeutung besitzen,
wenn man beispielsweise
a) Tetradepsipeptide der allgemeinen Formel (III) 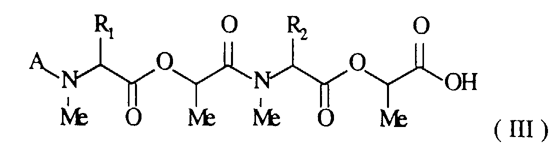 in welcher
   - A: für Wasserstoff oder Benzyl steht, oder für eine Gruppe der Formel - CO-R⁴ steht,
   worin
   - R⁴: für geradkettiges, verzweigtes Alkoxy oder Arylalkoxy mit bis zu 6 Kohlenstoffatomen
   im Alkylteil steht,
   - R¹ und R²: die oben angegebene Bedeutung haben,
   in einem ersten Reaktionsschritt mit Didepsipeptiden der allgemeinen Formel (VII) 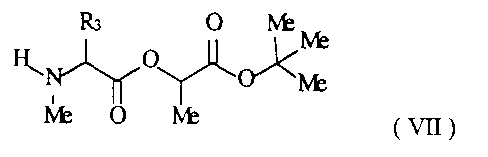 in welcher
   - R³: die weiter oben angegebene Bedeutung besitzt,
   in Gegenwart geeigneter Kupplungsreagenzien, in Gegenwart eines basischen Reaktionshilfsmittels und in Gegenwart eines Verdünnungsmittels umsetzt oder
b) Tetradepsipeptide der allgemeinen Formel (IV) 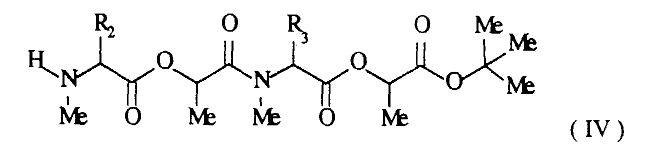 in welcher
   - R² und R³: die weiter oben angegebene Bedeutung besitzen,
   in einem ersten Reaktionsschritt mit Didepsipeptiden der allgemeinen Formel (V) 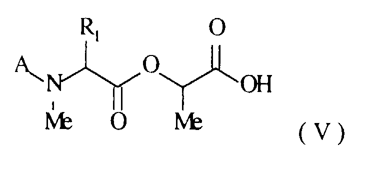 in welcher
   - A und R¹: die weiter oben angegebene Bedeutung besitzen,
   in Gegenwart geeigneter Kupplungsreagenzien, in Gegenwart eines basischen Reaktionshilfsmittels und in Gegenwart eines Verdünnungsmittels umsetzt,
   dann in einem zweiten Reaktionsschritt die aus den Verfahren a) oder b) resultierenden offenkettigen Hexadepsipeptide der allgemeinen Formel 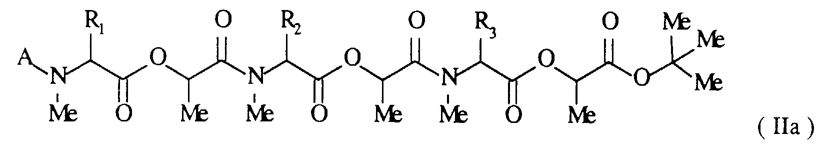 in welcher
   - A R¹, R² und R³: die oben angegebene Bedeutung haben,
   in Gegenwart eines Verdünnungsmittels und gegebenenfalls in Gegenwart einer Protonensäure C-terminal verseift oder in Gegenwart eines Verdünnungsmittels und gegebenenfalls in Gegenwart eines geeigneten Katalysators N-terminal deblockiert,
   dann in einem dritten Reaktionsschritt die resultierenden offenkettigen Hexadepsipeptide der allgemeinen Formel 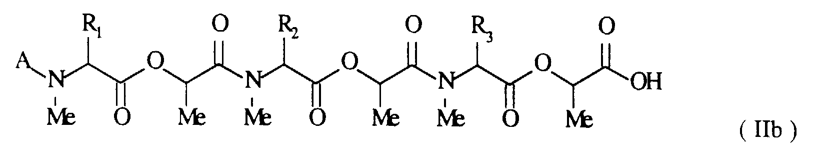 oder 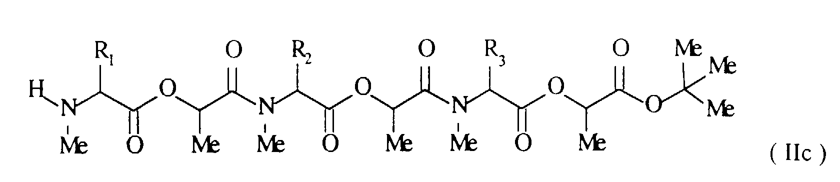 in welcher
   - A, R¹, R² und R³: die oben angegebene Bedeutung besitzen,
   in Gegenwart eines Verdünnungsmittels und gegebenenfalls in Gegenwart eines geeigneten Katalysators N-terminal deblockiert oder in Gegenwart eines Verdünnungsmittels und in Gegenwart einer Protonensäure erneut C-terminal verseift.

Die erfindungsgemäßen cyclischen Depsipeptide mit 18 Ringatomen sind durch die Formel (I) allgemein definiert.

Bevorzugt sind Verbindungen der Formel (I),
in welcher
- R: für Wasserstoff, geradkettiges oder verzweigtes C₁₋₈ -Alkyl, insbesondere Methyl, Ethyl, Propyl, Isopropyl, Butyl, Isobutyl, sec.-Butyl, tert.-Butyl, Pentyl, sec.-Pentyl, 1,2-Dimethyl-propyl, neo-Pentyl, 1-Ethyl-propyl, 1,1-Dimethylpropyl, Hexyl, Isohexyl, sec.-Hexyl, Heptyl, Isoheptyl, sec.-Heptyl, tert.-Heptyl, Oktyl, Isooktyl, sec.-Oktyl, C₃₋₇-Cycloalkyl, insbesondere Cyclopentyl, Cyclohexyl, Cycloheptyl, C₃₋₇-Cycloalkyl-C₁₋₄-alkyl, insbesondere Cyclopentylmethyl, Cyclohexylmethyl oder Cycloheptylmethyl steht.

Die Verbindungen der allgemeinen Formel (I) können 6 bis 8 Chiralitätszentren aufweisen.

Die optisch aktiven Verbindungen der allgemeinen Formel (I) besitzen mindestens eine festgelegte Konfiguration an einem der 6 bis 8 möglichen Chiralitätszentren, wobei an den restlichen Chiralitätszentren keine festgelegte Konfiguration vorliegen kann.

Besonders bevorzugt sind Verbindungen der allgemeinen Formel (I)
in welcher
- R: für Wasserstoff, Methyl, Ethyl, Propyl, Isopropyl, Butyl, Isobutyl oder sec.-Butyl steht,
Ganz besonders bevorzugt sind Verbindungen der allgemeinen Formel (I)
in welcher
- R: für Methyl, Ethyl oder sec.-Butyl steht, und deren stereoisomere Verbindungen die nachfolgende Bedeutung haben,
Cyclo(-N-methyl-L-alanyl-D-lactyl-N-methyl-D-isoleucyl-D-lactyl-N-methyl-L-alanyl-D-lactyl-),
Cyclo(-N-methyl-L-alanyl-D-lactyl-N-methyl-L-isoleucyl-D-lactyl-N-methyl-L-alanyl-D-lactyl-),
Cyclo(-N-methyl-L-alanyl-D-lactyl-N-methyl-L-alloisoleucyl-D-lactyl-N-methyl-L-alanyl-D-lactyl-),
Cyclo(-N-methyl-L-alanyl-D-lactyl-N-methyl-L-isoleucyl-L-lactyl-N-methyl-L-alanyl-D-lactyl-),
Cyclo(-N-methyl-L-alanyl-D-lactyl-N-methyl-D-isoleucyl-L-lactyl-N-methyl-L-alanyl-D-lactyl-),
Cyclo(-N-methylDalanyl-L-lactyl-N-methyl-D-isoleucyl-D-lactyl-N-methyl-D-alanyl-L-lactyl-),
Cyclo(-N-methyl-D-alanyl-L-lactyl-N-methyl-L-isoleucyl-L-lactyl-N-methyl-D-alanyl-L-lactyl-),
Cyclo(-N-methyl-D-alanyl-L-lactyl-N-methyl-L-isoleucyl-D-lactyl-N-methyl-D-alanyl-L-lactyl-),
Cyclo(-N-methyl-D-alanyl-L-lactyl-N-methyl-D-isoleucyl-L-lactyl-N-methyl-D-alanyl-L-lactyl-),
Cyclo(-N-methyl-D-alanyl-L-lactyl-N-methyl-D-alloisoleucyl-L-lactyl-N-methyl-D-alanyl-L-lactyl-),
Cyclo(-N-methyl-L-alanyl-D-lactyl-N-methyl-D-isoleucyl-D-lactyl-N-methyl-L-2-amino-butyryl-D-lactyl-),
Cyclo(-N-methyl-L-alanyl-D-lactyl-N-methyl-L-isoleucyl-D-lactyl-N-methyl-L-2-amino-butyryl-D-lactyl-),
Cyclo(-N-methyl-L-alanyl-D-lactyl-N-methyl-L-alloisoleucyl-D-lactyl-N-methyl-L-2-amino-butyryl-D-lactyl-),
Cyclo(-N-methyl-L-alanyl-D-lactyl-N-methyl-D-isoleucyl-L-lactyl-N-methyl-L-2-amino-butyryl-D-lactyl-),
Cyclo(-N-methyl-L-alanyl-D-lactyl-N-methyl-L-isoleucyl-D-lactyl-N-methyl-L-2-amino-butyryl-D-lactyl-),
Cyclo(-N-methyl-D-alanyl-L-lactyl-N-methyl-D-isoleucyl-D-lactyl-N-methyl-D-2-amino-butyryl-L-lactyl-),
Cyclo(-N-methyl-D-alanyl-L-lactyl-N-methyl-L-isoleucyl-L-lactyl-N-methyl-D-2-amino-butyry-L-lactyl-),
Cyclo(-N-methyl-D-alanyl-L-lactyl-N-methyl-L-isoleucyl-D-lactyl-N-methyl-D-2-amino-butyryl-L-lactyl-),
Cyclo(-N-methyl-D-alanyl-L-lactyl-N-methyl-D-isoleucyl-L-lactyl-N-methyl-D-2-amino-butyryl-L-lactyl-),
Cyclo(-N-methyl-D-alanyl-L-lactyl-N-methyl-D-alloisoleucy-L-lactyl-N-methyl-D-2-aminobutyryl-L-lactyl-),
Cyclo(-N-methyl-L-alanyl-D-lactyl-N-methyl-D-isoleucyl-D-lactyl-N-methyl-L-isoleucyl-D-lactyl-),
Cyclo(-N-methyl-L-alanyl-D-lactyl-N-methyl-L-isoleucyl-D-lactyl-N-methyl-L-isoleucyl-D-lactyl-),
Cyclo(-N-methyl-L-alanyl-D-lactyl-N-methyl-L-alloisoleucyl-D-lactyl-N-methyl-L-isoleucyl-D-lactyl-),
Cyclo(-N-methyl-L-alanyl-D-lactyl-N-methyl-L-isoleucyl-D-lactyl-N-methyl-L-alloisoleucyl-D-lactyl-),
Cyclo(-N-methyl-L-alanyl-D-lactyl-N-methyl-L-alloisoleucyl-D-lactyl-N-methyl-L-alloisoleucyl-D-lactyl-),
Cyclo(-N-methyl-L-alanyl-D-lactyl-N-methyl-L-isoleucyl-L-lactyl-N-methyl-L-isoleucyl-D-lactyl-),
Cyclo(-N-methyl-L-alanyl-D-lactyl-N-methyl-D-isoleucyl-L-lactyl-N-methyl-L-isoleucyl-D-lactyl-),
Cyclo(-N-methyl-D-alanyl-L-lactyl-N-methyl-D-isoleucyl-D-lactyl-N-methyl-D-isoleucyl-L-lactyl-),
Cyclo(-N-methyl-D-alanyl-L-lactyl-N-methyl-L-isoleucyl-L-lactyl-N-methyl-D-isoleucyl-L-lactyl-),
Cyclo(-N-methyl-D-alanyl-L-lactyl-N-methyl-L-isoleucyl-D-lactyl-N-methyl-D-isoleucyl-L-lactyl-),
Cyclo(-N-methyl-D-alanyl-L-lactyl-N-methyl-D-isoleucyl-L-lactyl-N-methyl-D-isoleucyl-L-lactyl-),
Cyclo(-N-methyl-D-alanyl-L-lactyl-N-methyl-D-alloisoleucyl-L-lactyl-N-methyl-D-isoleucyl-L-lactyl-),
Cyclo(-N-methyl-D-alanyl-L-lactyl-N-methyl-D-isoleucyl-L-lactyl-N-methyl-D-alloisoleucyl-L-lactyl-),
Cyclo(-N-methyl-D-alanyl-L-lactyl-N-methyl-D-alloisoleucyl-L-lactyl-N-methyl-D-alloisoleucyl-L-lactyl-).

Die Verbindungen der allgemeinen Formel (I) können in optisch aktiven, stereoisomeren Formen oder als racemische Gemische vorliegen. Bevorzugt sind jedoch optisch aktive, stereoisomere Formen der Verbindungen der allgemeinen Formel (I).

Im einzelnen seien folgende stereoisomeren Formen der Verbindungen der allgemeinen Formel (I) genannt:
Cyclo(-N-methyl-L-alanyl-D-lactyl-N-methyl-D-isoleucyl-D-lactyl-N-methyl-glycyl-D-lactyl-),
Cyclo(-N-methyl-L-alanyl-D-lactyl-N-methyl-L-isoleucyl-D-lactyl-N-methyl-glycyl-D-lactyl-),
Cyclo(-N-methyl-L-alanyl-D-lactyl-N-methyl-L-alloisoleucyl-D-lactyl-N-methyl-glycyl-D-lactyl-),
Cyclo(-N-methyl-L-alanyl-D-lactyl-N-methyl-L-isoleucyl-L-lactyl-N-methyl-glycyl-D-lactyl-),
Cyclo(-N-methyl-L-alanyl-D-lactyl-N-methyl-D-isoleucyl-L-lactyl-N-methyl-glycy-D-lactyl-),
Cyclo(-N-methyl-D-alanyl-L-lactyl-N-methyl-D-isoleucyl-D-lactyl-N-methyl-glycyl-L-lactyl-),
Cyclo(-N-methyl-D-alanyl-L-lactyl-N-methyl-L-isoleucyl-L-lactyl-N-methyl-glycyl-L-lactyl-),
Cyclo(-N-methyl-D-alanyl-L-lactyl-N-methyl-L-isoleucyl-D-lactyl-N-methyl-glycyl-L-lactyl-),
Cyclo(-N-methyl-D-alanyl-L-lactyl-N-methyl-D-isoleucyl-L-lactyl-N-methylglycyl-L-lactyl-),
Cyclo(-N-methyl-D-alanyl-L-lactyl-N-methyl-D-alloisoleucyl-L-lactyl-N-methyl-glycyl-L-lactyl-),
Cyclo(-N-methyl-L-alanyl-D-lactyl-N-methyl-D-isoleucyl-D-lactyl-N-methyl-D-alanyl-D-lactyl-),
Cyclo(-N-methyl-D-alanyl-L-lactyl-N-methyl-D-isoleucyl-D-lactyl-N-methyl-D-alanyl-D-lactyl-),
Cyclo(-N-methyl-D-alanyl-D-lactyl-N-methyl-L-isoleucyl-D-lactyl-N-methyl-D-alanyl-D-lactyl-),
Cyclo(-N-methyl-D-alanyl-D-lactyl-N-methyl-D-isoleucyl-L-lactyl-N-methyl-D-alanyl-D-lactyl-),
Cyclo(-N-methyl-D-alanyl-D-lactyl-N-methyl-D-isoleucyl-D-lactyl-N-methyl-L-alanyl-D-lactyl-),
Cyclo(-N-methyl-D-alanyl-D-lactyl-N-methyl-D-isoleucyl-D-lactyl-N-methyl-D-alanyl-L-lactyl-),
Cyclo(-N-methyl-D-alanyl-D-lactyl-N-methyl-D-isoleucyl-D-lactyl-N-methyl-D-alanyl-D-lactyl-),
Cyclo(-N-methyl-D-alanyl-L-lactyl-N-methyl-L-isoleucyl-L-lactyl-N-methyl-L-alanyl-L-lactyl-),
Cyclo(-N-methyl-L-alanyl-D-lactyl-N-methyl-L-isoleucyl-L-lactyl-N-methyl-L-alanyl-L-lacryl-),
Cyclo(-N-methyl-L-alanyl-L-lactyl-N-methyl-D-isoleucyl-L-lactyl-N-methyl-L-alanyl-L-lactyl-),
Cyclo(-N-methyl-L-alanyl-L-lactyl-N-methyl-L-isoleucyl-D-lactyl-N-methyl-L-alanyl-L-lactyl-),
Cyclo(-N-methyl-L-alanyl-L-lactyl-N-methyl-L-isoleucyl-L-lactyl-N-methyl-D-alanyl-L-lactyl-),
Cyclo(-N-methyl-L-alanyl-L-lactyl-N-methyl-L-isoleucyl-L-lactyl-N-methyl-L-alanyl-D-lactyl-),
Cyclo(-N-methyl-L-alanyl-L-lactyl-N-methyl-L-isoleucyl-L-lactyl-N-methyl-L-alanyl-L-lactyl-),
Cyclo(-N-methyl-L-alanyl-L-lactyl-N-methyl-D-isoleucyl-D-lactyl-N-methyl-D-alanyl-D-lactyl-),
Cyclo(-N-methyl-L-alanyl-D-lactyl-N-methyl-L-isoleucyl-D-lactyl-N-methyl-D-alanyl-D-lactyl-),
Cyclo(-N-methyl-L-alanyl-D-lactyl-N-methyl-D-isoleucyl-L-lactyl-N-methyl-D-alanyl-D-lactyl-),
Cyclo(-N-methyl-L-alanyl-D-lactyl-N-methyl-D-isoleucyl-D-lactyl-N-methyl-D-alanyl-L-lactyl-),
Cyclo(-N-methyl-D-alanyl-L-lactyl-N-methyl-L-isoleucyl-D-lactyl-N-methyl-D-alanyl-D-lactyl-),
Cyclo(-N-methyl-D-alanyl-L-lactyl-N-methyl-D-isoleucyl-L-lactyl-N-methyl-D-alanyl-D-lactyl-),
Cyclo(-N-methyl-D-alanyl-L-lactyl-N-methyl-D-isoleucyl-D-lactyl-N-methyl-L-alanyl-D-lactyl-),
Cyclo(-N-methyl-D-alanyl-D-lactyl-N-methyl-L-isoleucyl-L-lactyl-N-methyl-D-alanyl-D-lactyl-),
Cyclo(-N-methyl-D-alanyl-D-lactyl-N-methyl-L-isoleucyl-D-lactyl-N-methyl-L-alanyl-D-lactyl-),
Cyclo(-N-methyl-D-alanyl-D-lacryl-N-methyl-L-isoleucyl-D-lactyl-N-methyl-D-alanyl-L-lactyl-),
Cyclo(-N-methyl-D-alanyl-D-lactyl-N-methyl-D-isoleucyl-L-lactyl-N-methyl-L-alanyl-D-lactyl-),
Cyclo(-N-methyl-D-alanyl-D-lactyl-N-methyl-D-isoleucyl-L-lactyl-N-methyl-D-alanyl-L-lactyl-),
Cyclo(-N-methyl-D-alanyl-D-lactyl-N-methyl-D-isoleucyl-D-lactyl-N-methyl-L-alanyl-L-lactyl-),
Cyclo(-N-methyl-D-alanyl-D-lactyl-N-methyl-L-isoleucyl-L-lactyl-N-methyl-L-alanyl-L-lactyl-),
Cyclo(-N-methyl-D-alanyl-L-lactyl-N-methyl-D-isoleucyl-L-lactyl-N-methyl-L-alanyl-L-lactyl-),
Cyclo(-N-methyl-D-alanyl-L-lactyl-N-methyl-L-isoleucyl-D-lactyl-N-methyl-L-alanyl-L-lactyl-),
Cyclo(-N-methyl-D-alanyl-L-lactyl-N-methyl-L-isoleucyl-L-lactyl-N-methyl-L-alanyl-D-lactyl-),
Cyclo(-N-methyl-L-alanyl-D-lactyl-N-methyl-D-isoleucyl-L-lactyl-N-methyl-L-alanyl-L-lactyl-),
Cyclo(-N-methyl-L-alanyl-D-lactyl-N-methyl-L-isoleucyl-D-lactyl-N-methyl-L-alanyl-L-lactyl-),
Cyclo(-N-methyl-L-alanyl-D-lactyl-N-methyl-L-isoleucyl-L-lactyl-N-methyl-D-alanyl-L-lactyl-),
Cyclo(-N-methyl-L-alanyl-L-lactyl-N-methyl-D-isoleucyl-D-lactyl-N-methyl-L-alanyl-L-lactyl-),
Cyclo(-N-methyl-L-alanyl-L-lactyl-N-methyl-D-isoleucyl-L-lactyl-N-methyl-D-alanyl-L-lactyl-),
Cyclo(-N-methyl-L-alanyl-L-lactyl-N-methyl-D-isoleucyl-L-lactyl-N-methyl-L-alanyl-D-lactyl-),
Cyclo(-N-methyl-L-alanyl-L-lactyl-N-methyl-L-isoleucyl-D-lactyl-N-methyl-D-alanyl-L-lactyl-),
Cyclo(-N-methyl-L-alanyl-L-lactyl-N-methyl-L-isoleucyl-D-lactyl-N-methyl-L-alanyl-D-lactyl-),
Cyclo(-N-methyl-L-alanyl-L-lactyl-N-methyl-L-isoleucyl-L-lactyl-N-methyl-D-alanyl-D-lactyl-),
Cyclo(-N-methyl-L-alanyl-L-lactyl-N-methyl-L-isoleucyl-D-lactyl-N-methyl-D-alanyl-D-lactyl-),
Cyclo(-N-methyl-D-alanyl-L-lactyl-N-methyl-L-isoleucyl-L-lactyl-N-methyl-D-alanyl-D-lactyl-),
Cyclo(-N-methyl-D-alanyl-D-lactyl-N-methyl-L-isoleucyl-L-lactyl-N-methyl-L-alanyl-D-lactyl-),
Cyclo(-N-methyl-D-alanyl-D-lactyl-N-methyl-D-isoleucyl-L-lactyl-N-methyl-L-alanyl-L-lactyl-),
Cyclo(-N-methyl-L-alanyl-D-lactyl-N-methyl-L-isoleucyl-L-lactyl-N-methyl-D-alanyl-D-lactyl-),
Cyclo(-N-methyl-L-alanyl-D-lactyl-N-methyl-D-isoleucyl-D-lactyl-N-methyl-L-alanyl-L-lactyl-),
Cyclo(-N-methyl-L-alanyl-D-lactyl-N-methyl-L-isoleucyl-D-lactyl-N-methyl-D-alanyl-L-lactyl-),
Cyclo(-N-methyl-D-alanyl-L-lactyl-N-methyl-L-isoleucyl-D-lactyl-N-methyl-L-alanyl-D-lactyl-),
Cyclo(-N-methyl-D-alanyl-L-lactyl-N-methyl-D-isoleucyl-L-lactyl-N-methyl-L-alanyl-D-lactyl-),
Cyclo(-N-methyl-D-alanyl-L-lactyl-N-methyl-D-isoleucyl-D-lactyl-N-methyl-L-alanyl-L-lactyl-),
Cyclo(-N-methyl-D-alanyl-D-lactyl-N-methyl-L-isoleucyl-L-lactyl-N-methyl-D-alanyl-L-lactyl-),
Cyclo(-N-methyl-D-alanyl-D-lactyl-N-methyl-L-isoleucyl-D-lactyl-N-methyl-L-alanyl-L-lactyl-),
Cyclo(-N-methyl-L-alanyl-L-lactyl-N-methyl-D-isoleucyl-D-lactyl-N-methyl-D-alanyl-L-lactyl-),
Cyclo(-N-methyl-L-alanyl-D-lactyl-N-methyl-D-isoleucyl-L-lactyl-N-methyl-D-alanyl-L-lactyl-),
Cyclo(-N-methyl-L-alanyl-L-lactyl-N-methyl-D-isoleucyl-D-lactyl-N-methyl-L-alanyl-D-lactyl-),
Cyclo(-N-methyl-L-alanyl-L-lactyl-N-methyl-D-isoleucyl-L-lactyl-N-methyl-D-alanyl-D-lactyl-)
Cyclo(-N-methyl-L-alanyl-D-lactyl-N-methyl-D-isoleucyl-D-lactyl-N-methyl-D-alanyl-D-lactyl-),
Cyclo(-N-methyl-D-alanyl-L-lactyl-N-methyl-D-isoleucyl-D-lactyl-N-methyl-D-2-amino-butyryl-D-lactyl-),
Cyclo(-N-methyl-D-alanyl-D-lactyl-N-methyl-L-isoleucyl-D-lactyl-N-methyl-D-2-amino-butyryl-D-lactyl-),
Cyclo(-N-methyl-D-alanyl-D-lactyl-N-methyl-D-isoleucyl-L-lactyl-N-methyl-D-2-amino-butyryl-D-lactyl-),
Cyclo(-N-methyl-D-alanyl-D-lactyl-N-methyl-D-isoleucyl-D-lactyl-N-methyl-L-2-amino-butyryl-D-lactyl-),
Cyclo(-N-methyl-D-alanyl-D-lactyl-N-methyl-D-isoleucyl-D-lactyl-N-methyl-D-2-amino-butyryl-L-lactyl-),
Cyclo(-N-methyl-D-alanyl-D-lactyl-N-methyl-D-isoleucyl-D-lactyl-N-methyl-D-2-amino-butyryl-D-lactyl-),
Cyclo(-N-methyl-D-alanyl-L-lactyl-N-methyl-L-isoleucyl-L-lactyl-N-methyl-L-2-amino-butyryl-L-lactyl-),
Cyclo(-N-methyl-L-alanyl-D-lactyl-N-methyl-L-isoleucyl-L-lactyl-N-methyl-L-2-amino-butyryl-L-lactyl-),
Cyclo(-N-methyl-L-alanyl-L-lactyl-N-methyl-D-isoleucyl-L-lactyl-N-methyl-L-2-amino-butyryl-L-lactyl-),
Cyclo(-N-methyl-L-alanyl-L-lactyl-N-methyl-L-isoleucyl-D-lactyl-N-methyl-L-2-amino-butyryl-L-lactyl-),
Cyclo(-N-methyl-L-alanyl-L-lactyl-N-methyl-L-isoleucyl-L-lactyl-N-methyl-D-2-amino-butyryl-L-lactyl-),
Cyclo(-N-methyl-L-alanyl-L-lactyl-N-methyl-L-isoleucyl-L-lactyl-N-methyl-L-2-amino-butyryl-D-lactyl-),
Cyclo(-N-methyl-L-alanyl-L-lactyl-N-methyl-L-isoleucyl-L-lactyl-N-methyl-L-2-amino-butyryl-L-lactyl-),
Cyclo(-N-methyl-L-alanyl-L-lactyl-N-methyl-D-isoleucyl-D-lactyl-N-methyl-D-2-amino-butyryl-D-lactyl-),
Cyclo(-N-methyl-L-alanyl-D-lactyl-N-methyl-L-isoleucyl-D-lactyl-N-methyl-D-2-amino-butyryl-D-lactyl-),
Cyclo(-N-methyl-L-alanyl-D-lactyl-N-methyl-D-isoleucyl-L-lactyl-N-methyl-D-2-amino-butyryl-D-lactyl-),
Cyclo(-N-methyl-L-alanyl-D-lactyl-N-methyl-D-isoleucyl-D-lactyl-N-methyl-D-2-amino-butyryl-L-lactyl-),
Cyclo(-N-methyl-D-alanyl-L-lactyl-N-methyl-L-isoleucyl-D-lactyl-N-methyl-D-2-amino-butyryl-D-lactyl-),
Cyclo(-N-methyl-D-alanyl-L-lactyl-N-methyl-D-isoleucyl-L-lactyl-N-methyl-D-2-amino-butyryl-D-lactyl-),
Cyclo(-N-methyl-D-alanyl-L-lactyl-N-methyl-D-isoleucyl-D-lactyl-N-methyl-L-2-amino-butyryl-D-lactyl-),
Cyclo(-N-methyl-D-alanyl-D-lactyl-N-methyl-L-isoleucyl-L-lactyl-N-methyl-D-2-amino-butyryl-D-lactyl-),
Cyclo(-N-methyl-D-alanyl-D-lactyl-N-methyl-L-isoleucyl-D-lactyl-N-methyl-L-2-amino-butyryl-D-lactyl-),
Cyclo(-N-methyl-D-alanyl-D-lactyl-N-methyl-L-isoleucyl-D-lactyl-N-methyl-D-2-amino-butyryl-L-lactyl-),
Cyclo(-N-methyl-D-alanyl-D-lactyl-N-methyl-D-isoleucyl-L-lactyl-N-methyl-L-2-amino-butyryl-D-lactyl-),
Cyclo(-N-methyl-D-alanyl-D-lactyl-N-methyl-D-isoleucyl-L-lactyl-N-methyl-D-2-amino-butyryl-L-lactyl-),
Cyclo(-N-methyl-D-alanyl-D-lactyl-N-methyl-D-isoleucyl-D-lactyl-N-methyl-L-2-amino-butyryl-L-lactyl-),
Cyclo(-N-methyl-D-alanyl-D-lactyl-N-methyl-L-isoleucyl-L-lactyl-N-methyl-L-2-amino-butyryl-L-lactyl-),
Cyclo(-N-methyl-D-alanyl-L-lactyl-N-methyl-D-isoleucyl-L-lactyl-N-methyl-L-2-amino-butyryl-L-lactyl-),
Cyclo(-N-methyl-D-alanyl-L-lactyl-N-methyl-L-isoleucyl-D-lactyl-N-methyl-L-2-amino-butyryl-L-lactyl-),
Cyclo(-N-methyl-D-alanyl-L-lactyl-N-methyl-L-isoleucyl-L-lactyl-N-methyl-L-2-amino-butyryl-D-lactyl-),
Cyclo(-N-methyl-L-alanyl-D-lactyl-N-methyl-D-isoleucyl-L-lactyl-N-methyl-L-2-amino-butyryl-L-lactyl-),
Cyclo(-N-methyl-L-alanyl-D-lactyl-N-methyl-L-isoleucyl-D-lactyl-N-methyl-L-2-amino-butyryl-L-lactyl-),
Cyclo(-N-methyl-L-alanyl-D-lactyl-N-methyl-L-isoleucyl-L-lactyl-N-methyl-D-2-amino-butyryl-L-lactyl-),
Cyclo(-N-methyl-L-alanyl-L-lactyl-N-methyl-D-isoleucyl-D-lactyl-N-methyl-L-2-amino-butyryl-L-lactyl-),
Cyclo(-N-methyl-L-alanyl-L-lactyl-N-methyl-D-isoleucyl-L-lactyl-N-methyl-D-2-amino-butyryl-L-lactyl-),
Cyclo(-N-methyl-L-alanyl-L-lactyl-N-methyl-D-isoleucyl-L-lactyl-N-methyl-L-2-amino-butyryl-D-lactyl-),
Cyclo(-N-methyl-L-alanyl-L-lactyl-N-methyl-L-isoleucyl-D-lactyl-N-methyl-D-2-amino-butyryl-L-lactyl-),
Cyclo(-N-methyl-L-alanyl-L-lactyl-N-methyl-L-isoleucyl-D-lactyl-N-methyl-L-2-amino-butyryl-D-lactyl-),
Cyclo(-N-methyl-L-alanyl-L-lactyl-N-methyl-L-isoleucyl-L-lactyl-N-methyl-D-2-amino-butyryl-D-lactyl-),
Cyclo(-N-methyl-L-alanyl-L-lactyl-N-methyl-L-isoleucyl-D-lactyl-N-methyl-D-2-amino-butyryl-D-lactyl-),
Cyclo(-N-methyl-D-alanyl-L-lactyl-N-methyl-L-isoleucyl-L-lactyl-N-methyl-D-2-amino-butyryl-D-lactyl-),
Cyclo(-N-methyl-D-alanyl-D-lactyl-N-methyl-L-isoleucyl-L-lactyl-N-methyl-L-2-amino-butyryl-D-lactyl-),
Cyclo(-N-methyl-D-alanyl-D-lactyl-N-methyl-D-isoleucyl-L-lactyl-N-methyl-L-2-amino-butyryl-L-lactyl-),
Cyclo(-N-methyl-L-alanyl-D-lactyl-N-methyl-L-isoleucyl-L-lactyl-N-methyl-D-2-amino-butyryl-D-lactyl-),
Cyclo(-N-methyl-L-alanyl-D-lactyl-N-methyl-D-isoleucyl-D-lactyl-N-methyl-L-2-amino-butyryl-L-lactyl-),
Cyclo(-N-methyl-L-alanyl-D-lactyl-N-methyl-L-isoleucyl-D-lactyl-N-methyl-D-2-amino-butyryl-L-lactyl-),
Cyclo(-N-methyl-D-alanyl-L-lactyl-N-methyl-L-isoleucyl-D-lactyl-N-methyl-L-2-amino-butyryl-D-lactyl-),
Cyclo(-N-methyl-D-alanyl-L-lactyl-N-methyl-D-isoleucyl-L-lactyl-N-methyl-L-2-amino-butyryl-D-lactyl-),
Cyclo(-N-methyl-D-alanyl-L-lactyl-N-methyl-D-isoleucyl-D-lactyl-N-methyl-L-2-amino-butyryl-L-lactyl-),
Cyclo(-N-methyl-D-alanyl-D-lactyl-N-methyl-L-isoleucyl-L-lactyl-N-methyl-D-2-amino-butyryl-L-lactyl-),
Cyclo(-N-methyl-D-alanyl-D-lactyl-N-methyl-L-isoleucyl-D-lactyl-N-methyl-L-2-amino-butyryl-L-lactyl-),
Cyclo(-N-methyl-L-alanyl-L-lactyl-N-methyl-D-isoleucyl-D-lactyl-N-methyl-D-2-amino-butyryl-L-lactyl-),
Cyclo(-N-methyl-L-alanyl-D-lactyl-N-methyl-D-isoleucyl-L-lactyl-N-methyl-D-2-amino-butyryl-L-lactyl-),
Cyclo(-N-methy-L-alanyl-L-lactyl-N-methyl-D-isoleucyl-D-lactyl-N-methyl-L-2-amino-butyryl-D-lactyl-),
Cyclo(-N-methyl-L-alanyl-L-lactyl-N-methyl-D-isoleucyl-L-lactyl-N-methyl-D-2-amino-butyryl-D-lactyl-),
Cyclo(-N-methyl-L-alanyl-D-lactyl-N-methyl-D-isoleucyl-D-lactyl-N-methyl-L-norvalyl-D-lactyl-),
Cyclo(-N-methyl-L-alanyl-D-lactyl-N-methyl-L-isoleucyl-D-lactyl-N-methyl-L-norvalyl-D-lactyl-),
Cyclo(-N-methyl-L-alanyl-D-lactyl-N-methyl-L-alloisoleucyl-D-lactyl-N-methy-L-norvalyl-D-lactyl-),
Cyclo(-N-methyl-L-alanyl-D-lactyl-N-methyl-L-isoleucyl-L-lactyl-N-methyl-L-norvalyl-D-lactyl-),
Cyclo(-N-methyl-L-alanyl-D-lactyl-N-methyl-D-isoleucyl-L-lactyl-N-methyl-L-norvalyl-D-lactyl-),
Cyclo(-N-methyl-D-alanyl-L-lactyl-N-methyl-D-isoleucyl-D-lactyl-N-methyl-D-norvalyl-L-lactyl-),
Cyclo(-N-methyl-D-alanyl-L-lactyl-N-methyl-L-isoleucyl-L-lactyl-N-methyl-D-norvalyl-L-lactyl-),
Cyclo(-N-methyl-D-alanyl-L-lactyl-N-methyl-L-isoleucyl-D-lactyl-N-methyl-D - norvalyl-L-lactyl-),
Cyclo(-N-methyl-D-alanyl-L-lactyl-N-methyl-D-isoleucyl-L-lactyl-N-methyl-D-norvalyl-L-lactyl-),
Cyclo(-N-methyl-D-alanyl-L-lactyl-N-methyl-D-alloisoleucyl-L-lactyl-N-methyl-D-norvalyl-L-lactyl-),
Cyclo(-N-methyl-L-alanyl-D-lactyl-N-methyl-D-isoleucyl-D-lactyl-N-methyl-L-valyl-D-lactyl-),
Cyclo(-N-methyl-L-alanyl-D-lactyl-N-methyl-L-isoleucyl-D-lactyl-N-methyl-L-valyl-D-lactyl-),
Cyclo(-N-methyl-L-alanyl-D-lactyl-N-methyl-L-alloisoleucyl-D-lactyl-N-methyl-L-valyl-D-lactyl-),
Cyclo(-N-methyl-L-alanyl-D-lactyl-N-methyl-L-isoleucyl-L-lactyl-N-methyl-L-valyl-D-lactyl-),
Cyclo(-N-methyl-L-alanyl-D-lactyl-N-methyl-D-isoleucyl-L-lactyl-N-methyl-L-valyl-D-lactyl-),
Cyclo(-N-methyl-D-alanyl-L-lactyl-N-methyl-D-isoleucyl-D-lactyl-N-methyl-D-valyl-L-lactyl-),
Cyclo(-N-methyl-D-alanyl-L-lactyl-N-methyl-L-isoleucyl-L-lactyl-N-methyl-D-valyl-L-lactyl-),
Cyclo(-N-methyl-D-alanyl-L-lactyl-N-methyl-L-isoleucyl-D-lactyl-N-methyl-D-valyl-L-lactyl-),
Cyclo(-N-methyl-D-alanyl-L-lactyl-N-methyl-D-isoleucyl-L-lactyl-N-methyl-D-valyl-L-lactyl-),
Cyclo(-N-methyl-D-alanyl-L-lactyl-N-methyl-D-alloisoleucyl-L-lactyl-N-methyl-D-valyl-L-lactyl-),
Cyclo(-N-methyl-L-alanyl-D-lactyl-N-methyl-D-isoleucyl-D-lactyl-N-methyl-L-norleucyl-D-lactyl-),
Cyclo(-N-methyl-L-alanyl-D-lactyl-N-methyl-L-isoleucyl-D-lactyl-N-methyl-L-norleucyl-D-lactyl-),
Cyclo(-N-methyl-L-alanyl-D-lactyl-N-methyl-L-alloisoleucyl-D-lactyl-N-methyl-L-norleucyl-D-lactyl-),
Cyclo(-N-methyl-L-alanyl-D-lactyl-N-methyl-L-isoleucyl-L-lactyl-N-methyl-L-norleucyl-D-lactyl-),
Cyclo(-N-methyl-L-alanyl-D-lactyl-N-methyl-D-isoleucyl-L-lactyl-N-methyl-L-norleucyl-D-lactyl-),
Cyclo(-N-methyl-D-alanyl-L-lactyl-N-methyl-D-isoleucyl-D-lactyl-N-methyl-D-norleucyl-L-lactyl-),
Cyclo(-N-methyl-D-alanyl-L-lactyl-N-methyl-L-isoleucyl-L-lactyl-N-methyl-D-norleucyl-L-lactyl-),
Cyclo(-N-methyl-D-alanyl-L-lactyl-N-methyl-L-isoleucyl-D-lactyl-N-methyl-D-norleucyl-L-lactyl-),
Cyclo(-N-methyl-D-alanyl-L-lactyl-N-methyl-D-isoleucyl-L-lactyl-N-methyl-D-norleucyl-L-lactyl-),
Cyclo(-N-methyl-D-alanyl-L-lactyl-N-methyl-D-alloisoleucyl-L-lactyl-N-methyl-D-norleucyl-L-lactyl-),
Cyclo(-N-methyl-L-alanyl-D-lactyl-N-methyl-D-isoleucyl-D-lactyl-N-methyl-L-leucyl-D-lactyl-),
Cyclo(-N-methyl-L-alanyl-D-lactyl-N-methyl-L-isoleucyl-D-lactyl-N-methyl-L-leucyl-D-lactyl-),
Cyclo(-N-methyl-L-alanyl-D-lactyl-N-methyl-L-alloisoleucyl-D-lactyl-N-methyl-L-leucyl-D-lactyl-),
Cyclo(-N-methyl-L-alanyl-D-lactyl-N-methyl-L-isoleucyl-L-lactyl-N-methy-L-leucyl-D-lactyl-),
Cyclo(-N-methyl-L-alanyl-D-lactyl-N-methyl-D-isoleucyl-L-lactyl-N-methyl-L-leucyl-D-lactyl-),
Cyclo(-N-methyl-D-alanyl-L-lactyl-N-methyl-D-isoleucyl-D-lactyl-N-methyl-D-leucyl-L-lactyl-),
Cyclo(-N-methyl-D-alanyl-L-lactyl-N-methyl-L-isoleucy-L-lactyl-N-methyl-D-leucyl-L-lactyl-),
Cyclo(-N-methyl-D-alanyl-L-lactyl-N-methyl-L-isoleucyl-D-lactyl-N-methyl-D-leucyl-L-lactyl-),
Cyclo(-N-methyl-D-alanyl-L-lactyl-N-methyl-D-isoleucyl-L-lactyl-N-methyl-D-leucyl-L-lactyl-),
Cyclo(-N-methyl-D-alanyl-L-lactyl-N-methyl-D-alloisoleucyl-L-lactyl-N-methyl-D-leucyl-L-lactyl-),
Cyclo(-N-methyl-L-alanyl-D-lactyl-N-methyl-D-isoleucyl-D-lactyl-N-methyl-D-isoleucyl-D-lactyl-),
Cyclo(-N-methyl-D-alanyl-L-lactyl-N-methyl-D-isoleucyl-D-lactyl-N-methyl-D-isoleucyl-D-lactyl-),
Cyclo(-N-methyl-D-alanyl-D-lactyl-N-methyl-L-isoleucyl-D-lactyl-N-methyl-D-isoleucyl-D-lactyl-),
Cyclo(-N-methyl-D-alanyl-D-lactyl-N-methyl-D-isoleucy-L-lactyl-N-methyl-D-isoleucyl-D-lactyl-),
Cyclo(-N-methyl-D-alanyl-D-lactyl-N-methyl-D-isoleucyl-D-lactyl-N-methyl-L-isoleucyl-D-lactyl-),
Cyclo(-N-methyl-D-alanyl-D-lactyl-N-methyl-D-isoleucyl-D-lactyl-N-methyl-D-isoleucyl-L-lactyl-),
Cyclo(-N-methyl-D-alanyl-D-lactyl-N-methyl-D-isoleucyl-D-lactyl-N-methyl-D-isoleucyl-D-lactyl-),
Cyclo(-N-methyl-D-alanyl-L-lactyl-N-methyl-L-isoleucyl-L-lactyl-N-methyl-L-isoleucyl-L-lactyl-),
Cyclo(-N-methyl-L-alanyl-D-lactyl-N-methyl-L-isoleucyl-L-lactyl-N-methyl-L-isoleucyl-L-lactyl-),
Cyclo(-N-methyl-L-alanyl-L-lactyl-N-methyl-D-isoleucyl-L-lactyl-N-methyl-L-isoleucyl-L-lactyl-),
Cyclo(-N-methyl-L-alanyl-L-lactyl-N-methyl-L-isoleucyl-D-lactyl-N-methyl-L-isoleucyl-L-lactyl-),
Cyclo(-N-methyl-L-alanyl-L-lactyl-N-methyl-L-isoleucyl-L-lactyl-N-methyl-D-isoleucyl-L-lactyl-),
Cyclo(-N-methyl-L-alanyl-L-lactyl-N-methyl-L-isoleucyl-L-lactyl-N-methyl-L-isoleucyl-D-lactyl-),
Cyclo(-N-methyl-L-alanyl-L-lactyl-N-methyl-L-isoleucyl-L-lactyl-N-methyl-L-isoleucyl-L-lactyl-),
Cyclo(-N-methyl-L-alanyl-L-lactyl-N-methyl-D-isoleucyl-D-lactyl-N-methyl-D-isoleucyl-D-lactyl-),
Cyclo(-N-methyl-L-alanyl-D-lactyl-N-methyl-L-isoleucyl-D-lactyl-N-methyl-D-isoleucyl-D-lactyl-),
Cyclo(-N-methyl-L-alanyl-D-lactyl-N-methyl-D-isoleucyl-L-lactyl-N-methyl-D-isoleucyl-D-lactyl-),
Cyclo(-N-methyl-L-alanyl-D-lactyl-N-methyl-D-isoleucyl-D-lactyl-N-methyl-D-isoleucyl-L-lactyl-),
Cyclo(-N-methyl-D-alanyl-L-lactyl-N-methyl-L-isoleucyl-D-lactyl-N-methyl-D-isoleucyl-D-lactyl-),
Cyclo(-N-methyl-D-alanyl-L-lactyl-N-methyl-D-isoleucyl-L-lactyl-N-methyl-D-isoleucyl-D-lactyl-),
Cyclo(-N-methyl-D-alanyl-L-lactyl-N-methyl-D-isoleucyl-D-lactyl-N-methyl-L-alanyl-D-lactyl-),
Cyclo(-N-methyl-D-alanyl-D-lactyl-N-methyl-L-isoleucyl-L-lactyl-N-methyl-D-isoleucyl-D-lactyl-),
Cyclo(-N-methyl-D-alanyl-D-lactyl-N-methyl-L-isoleucyl-D-lactyl-N-methyl-L-isoleucyl-D-lactyl-),
Cyclo(-N-methyl-D-alanyl-D-lactyl-N-methyl-L-isoleucyl-D-lactyl-N-methyl-D-isoleucyl-L-lactyl-),
Cyclo(-N-methyl-D-alanyl-D-lactyl-N-methyl-D-isoleucyl-L-lactyl-N-methyl-L-isoleucyl-D-lactyl-),
Cyclo(-N-methyl-D-alanyl-D-lactyl-N-methyl-D-isoleucyl-L-lactyl-N-methyl-D-isoleucyl-L-lactyl-),
Cyclo(-N-methyl-D-alanyl-D-lactyl-N-methyl-D-isoleucyl-D-lactyl-N-methyl-L-isoleucyl-L-lactyl-),
Cyclo(-N-methyl-D-alanyl-D-lactyl-N-methyl-L-isoleucyl-L-lactyl-N-methyl-L-isoleucyl-L-lactyl-),
Cyclo(-N-methyl-D-alanyl-L-lactyl-N-methyl-D-isoleucyl-L-lactyl-N-methyl-L-isoleucyl-L-lactyl-),
Cyclo(-N-methyl-D-alanyl-L-lactyl-N-methyl-L-isoleucyl-D-lactyl-N-methyl-L-isoleucyl-L-lactyl-),
Cyclo(-N-methyl-D-alanyl-L-lactyl-N-methyl-L-isoleucyl-L-lactyl-N-methyl-L-isoleucyl-D-lactyl-),
Cyclo(-N-methyl-L-alanyl-D-lactyl-N-methyl-D-isoleucyl-L-lactyl-N-methyl-L-isoleucyl-L-lactyl-),
Cyclo(-N-methyl-L-alanyl-D-lactyl-N-methyl-L-isoleucyl-D-lactyl-N-methyl-L-isoleucyl-L-lactyl-),
Cyclo(-N-methyl-L-alanyl-D-lactyl-N-methyl-L-isoleucyl-L-lactyl-N-methyl-D-isoleucyl-L-lactyl-),
Cyclo(-N-methyl-L-alanyl-L-lactyl-N-methyl-D-isoleucyl-D-lactyl-N-methyl-L-isoleucyl-L-lactyl-),
Cyclo(-N-methyl-L-alanyl-L-lactyl-N-methyl-D-isoleucyl-L-lactyl-N-methyl-D-isoleucyl-L-lactyl-),
Cyclo(-N-methyl-L-alanyl-L-lactyl-N-methyl-D-isoleucyl-L-lactyl-N-methyl-L-isoleucyl-D-lactyl-),
Cyclo(-N-methyl-L-alanyl-L-lactyl-N-methyl-L-isoleucyl-D-lactyl-N-methyl-D-isoleucyl-L-lactyl-),
Cyclo(-N-methyl-L-alanyl-L-lactyl-N-methyl-L-isoleucyl-D-lactyl-N-methyl-L-isoleucyl-D-lactyl-),
Cyclo(-N-methyl-L-alanyl-L-lactyl-N-methyl-L-isoleucyl-L-lactyl-N-methyl-D-isoleucyl-D-lactyl-),
Cyclo(-N-methyl-L-alanyl-L-lactyl-N-methyl-L-isoleucyl-D-lactyl-N-methyl-D-isoleucyl-D-lactyl-),
Cyclo(-N-methyl-D-alanyl-L-lactyl-N-methyl-L-isoleucyl-L-lactyl-N-methyl-D-isoleucyl-D-lactyl-),
Cyclo(-N-methyl-D-alanyl-D-lactyl-N-methyl-L-isoleucyl-L-lactyl-N-methyl-L-isoleucyl-D-lactyl-),
Cyclo(-N-methyl-D-alanyl-D-lactyl-N-methyl-D-isoleucyl-L-lactyl-N-methyl-L-isoleucyl-L-lactyl-),
Cyclo(-N-methyl-L-alanyl-D-lactyl-N-methyl-L-isoleucyl-L-lactyl-N-methyl-D-isoleucyl-D-lactyl-),
Cyclo(-N-methyl-L-alanyl-D-lactyl-N-methyl-D-isoleucyl-D-lactyl-N-methyl-L-isoleucyl-L-lactyl-),
Cyclo(-N-methyl-L-alanyl-D-lactyl-N-methyl-L-isoleucyl-D-lactyl-N-methyl-D-isoleucyl-L-lactyl-),
Cyclo(-N-methyl-D-alanyl-L-lactyl-N-methyl-L-isoleucyl-D-lactyl-N-methyl-L-isoleucyl-D-lactyl-),
Cyclo(-N-methyl-D-alanyl-L-lactyl-N-methyl-D-isoleucyl-L-lactyl-N-methyl-L-isoleucyl-D-lactyl-),
Cyclo(-N-methyl-D-alanyl-L-lactyl-N-methyl-D-isoleucyl-D-lactyl-N-methyl-L-isoleucyl-L-lactyl-),
Cyclo(-N-methyl-D-alanyl-D-lactyl-N-methyl-L-isoleucyl-L-lactyl-N-methyl-D-isoleucyl-L-lactyl-),
Cyclo(-N-methyl-D-alanyl-D-lactyl-N-methyl-L-isoleucyl-D-lactyl-N-methyl-L-isoleucyl-L-lactyl-),
Cyclo(-N-methyl-L-alanyl-L-lactyl-N-methyl-D-isoleucyl-D-lactyl-N-methyl-D-isoleucyl-L-lactyl-),
Cyclo(-N-methyl-L-alanyl-D-lactyl-N-methyl-D-isoleucyl-L-lactyl-N-methyl-D-isoleucyl-L-lactyl-),
Cyclo(-N-methyl-L-alanyl-L-lactyl-N-methyl-D-isoleucyl-D-lactyl-N-methyl-L-isoleucyl-D-lactyl-),
Cyclo(-N-methyl-L-alanyl-L-lactyl-N-methyl-D-isoleucyl-L-lactyl-N-methyl-D-isoleucyl-D-lactyl-)
Cyclo(-N-methyl-L-alanyl-D-lactyl-N-methyl-D-isoleucyl-D-lactyl-N-methyl-D-isoleucyl-D-lactyl-).

Setzt man beispielsweise nach Verfahren 2 zur Herstellung der neuen cyclischen Hexadepsipeptide der Formel (I) als Verbindungen der Formel (II) N-Methyl-L-alanyl-D-lactyl-N-methyl-L-isoleucyl-D-lactyl-N-methyl-L-isoleucyl-D-milchsäure ein, so läßt sich das Verfahren durch folgendes Reaktionsschema wiedergeben:

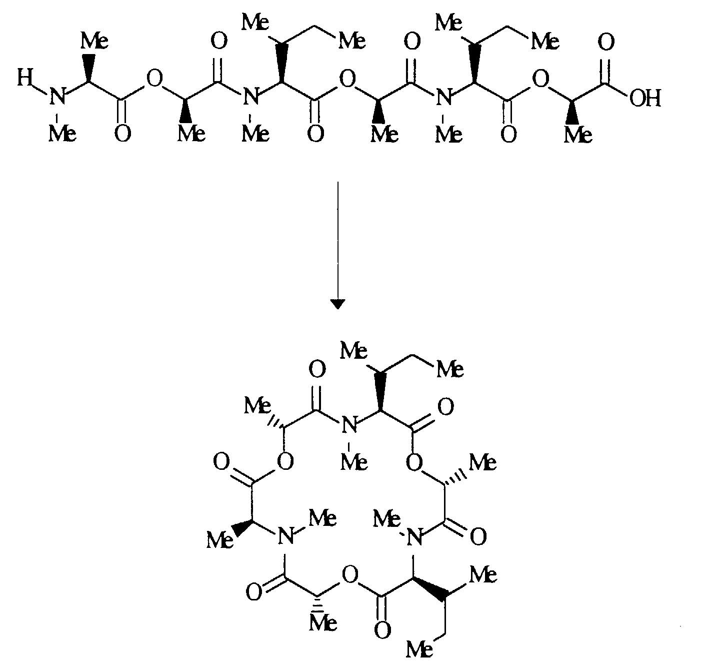

Die zur Durchführung des Verfahrens als Ausgangsstoffe benötigten offenkettigen Hexadepsipeptide sind durch die Formel (II) allgemein definiert. In dieser Formel stehen R¹ bis R³ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten offenkettigen Hexadepsipeptide der Formel (II) sind in einer vorgängigen, jedoch nicht vorveröffentlichten Patentanmeldung beschrieben (vgl. Deutsche Patentanmeldung P 4 317 458.2). 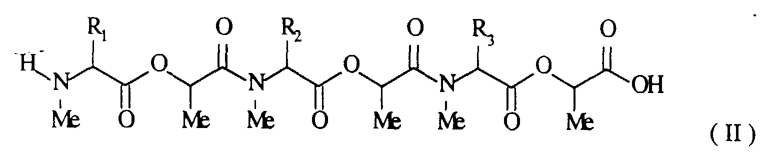 Im einzelnen seien folgende stereoisomeren Verbindungen der allgemeinen Formel (II) genannt:
N-Methyl-L-alanyl-D-lactyl-N-methyl-L-isoleucyl-D-lactyl-N-methyl-glycyl-D-milchsäure,
N-Methyl-L-alanyl-D-lactyl-N-methyl-L-alloisoleucyl-D-lactyl-N-methyl-glycyl-D-milchsäure,
N-Methyl-L-isoleucyl-D-lactyl-N-methyl-glycyl-D-lactyl-N-methyl-L-alanyl-D-milchsäure,
N-Methyl-L-alloisoleucyl-D-lactyl-N-methyl-glycyl-D-lactyl-N-methyl-L-alanyl-D-milchsäure,
N-Methyl-glycyl-D-lactyl-N-methyl-L-alanyl-D-lactyl-N-methyl-L-alloisoleucyl-D-milchsäure,
N-Methyl-glycyl-D-lactyl-N-methyl-D-alanyl-D-lactyl-N-methyl-L-alloisoleucyl-D-milchsäure,
N-Methyl-glycyl-L-lactyl-N-methvl-D-alanyl-L-lactyl-N-methyl-D-alloisoleucyl-L-milchsäure,
N-Methyl-L-alanyl-D-lactyl-N-methyl-D-isoleucyl-D-lactyl-N-methyl-L-alanyl-D-milchsäure,
N-Methyl-L-alanyl-D-lactyl-N-methyl-L-alloisoleucyl-D-lactyl-N-methyl-L-alanyl-D-milchsäure,
N-Methyl-L-alanyl-D-lactyl-N-methyl-L-isoleucyl-L-lactyl-N-methyl-L-alanyl-D-milchsäure,
N-Methyl-L-alanyl-D-lactyl-N-methyl-D-isoleucyl-L-lactyl-N-methyl-L-alanyl-D-milchsäure,
N-Methyl-D-alanyl-L-lactyl-N-methyl-D-isoleucyl-D-lactyl-N-methyl-D-alanyl-L-milchsäure,
N-Methyl-D-alanyl-L-lacryl-N-methyl-L-isoleucyl-L-lactyl-N-methyl-D-alanyl-L-milchsäure
N-Methyl-D-alanyl-L-lactyl-N-methyl-L-isoleucyl-D-lactyl-N-methyl-D-alanyl-L-milchsäure,
N-Methyl-D-alanyl-L-lactyl-N-methyl-D-isoleucyl-L-lactyl-N-methyl-D-alanyl-L-milchsäure,
N-Methyl-D-alanyl-L-lactyl-N-methyl-D-alloisoleucyl-L-lactyl-N-methyl-D-alanyl-L-milchsäure,
N-Methyl-L-2-aminobutyryl-D-lactyl-N-methyl-D-alanyl-D-lactyl-N-methyl-L-isoleucyl-D-milchsäure,
N-Methyl-L-alanyl-D-lactyl-N-methyl-L-isoleucyl-D-lactyl-N-methyl-L-2 aminobutyryl-D-milchsäure,
N-Methyl-L-isoleucyl-D-lactyl-N-methyl-L-2-aminobutyryl-D-lactyl-N-methyl-L-alanyl-D-milchsäure,
N-Methyl-L-2-aminobutyryl-D-lactyl-N-methyl-L-alanyl-D-lactyl-N-methyl-L-alloisoleucyI-D-milchsäure,
N-Methyl-L-2-aminobutyryl-D-lactyl-N-methyl-L-alanyl-D-lactyl-N-methyl-D-isoleucyl-L-milchsäure,
N-Methyl-L-2-aminobutyryl-D-lactyl-N-methyl-L-alanyl-D-lactyl-N-methyl-L-isoleucyl-D-milchsäure,
N-Methyl-D-2-aminobutyryl-L-lactyl-N-methyl-D-alanyl-D-lactyl-N-methyl-D-isoleucyl-L-milchsäure,
N-Methyl-D-2-aminobutyryl-L-lactyl-N-methyl-D-alanyl-L-lactyl-N-methyl-L-isoleucyl-L-milchsäure,
N-Methyl-D-2-aminobutyryl-L-lactyl-N-methyl-D-alanyl-L-lactyl-N-methyl-L-isoleucyl-D-milchsäure,
N-Methyl-D-2-aminobutyryl-L-lactyl-N-methyl-D-alanyl-L-lactyl-N-methyl-D-isoleucyl-L-milchsäure,
N-Methyl-D-2-aminobutyryl-L-lactyl-N-methyl-D-alanyl-L-lactyl-N-methyl-D-alloisoleucyl-L-milchsäure,
N-Methyl-L-norvalyl-D-lactyl-N-methyl-D-alanyl-D-lactyl-N-methyl-L-isoleucyl-D-milchsäure,
N-Methyl-L-alanyl-D-lactyl-N-methyl-L-isoleucyl-D-lactyl-N-methyl-L-norvalyl-D-milchsäure,
N-Methyl-L-isoleucyl-D-lactyl-N-methyl-L-norvalyl-D-lactyl-N-methyl-L-alanyl-D-milchsäure,
N-Methyl-L-norvalyl-D-lactyl-N-methyl-L-alanyl-D-lactyl-N-methyl-L-alloisoleucyl-D-milchsäure,
N-Methy-L-norvalyl-D-lactyl-N-methyl-L-alanyl-D-lactyl-N-methyl-D-isoleucyl-L-milchsäure,
N-Methyl-L-norvalyl-D-lactyl-N-methyl-L-alanyl-D-lactyl-N-methyl-L-isoleucyl-D-milchsäure,
N-Methyl-D-norvalyl-L-lactyl-N-methyl-D-alanyl-D-lactyl-N-methyl-D-isoleucyl-L-milchsäure,
N-Methyl-D-norvalyl-L-lactyl-N-methyl-D-alanyl-L-lactyl-N-methyl-L-isoleucyl-L-milchsäure,
N-Methyl-D-norvalyl-L-lactyl-N-methyl-D-alanyl-L-lactyl-N-methyl-L-isoleucyl-D-milchsäure,
N-Methyl-D-norvalyl-L-lactyl-N-methyl-D-alanyl-L-lactyl-N-methyl-D-isoleucyl-L-milchsäure,
N-Methyl-D-norvalyl-L-lactyl-N-methyl-D-alanyl-L-lactyl-N-methyl-D-alloisoleucyl-L-milchsäure,
N-Methyl-L-valyl-D-lactyl-N-methyl-D-alanyl-D-lactyl-N-methyl-L-isoleucyl-D-milchsäure,
N-Methyl-L-alanyl-D-lactyl-N-methyl-L-isoleucyl-D-lactyl-N-methyl-L-valyl-D-milchsäure,
N-Methyl-L-isoleucyl-D-lactyl-N-methyl-L-valyl-D-lactyl-N-methyl-L-alanyl-D-milchsäure,
N-Methyl-L-valyl-D-lactyl-N-methyl-L-alanyl-D-lactyl-N-methyl-L-alloisoleucyl-D-milchsäure,
N-Methyl-L-valyl-D-lactyl-N-methyl-L-alanyl-D-lactyl-N-methyl-D-isoleucyl-L-milchsäure,
N-Methyl-L-valyl-D-lactyl-N-methyl-L-alanyl-D-lactyl-N-methyl-L-isoleucyl-D-milchsäure,
N-Methyl-D-valyl-L-lactyl-N-methyl-D-alanyl-D-lactyl-N-methyl-D-isoleucyl-L-milchsäure,
N-Methyl-D-valyl-L-lactyl-N-methyl-D-alanyl-L-lactyl-N-methyl-L-isoleucyl-L-milchsäure,
N-Methyl-D-valyl-L-lactyl-N-methyl-D-alanyl-L-lactyl-N-methyl-L-isoleucyl-D-milchsäure,
N-Methyl-D-valyl-L-lactyl-N-methyl-D-alanyl-L-lactyl-N-methyl-D-isoleucyl-L-milchsäure,
N-Methyl-D-valyl-L-lactyl-N-methyl-D-alanyl-L-lactyl-N-methyl-D-alloisoleucyl-L-milchsäure,
N-Methyl-L-norleucyl-D-lactyl-N-methyl-D-alanyl-D-lactyl-N-methyl-L-isoleucyl-D-milchsäure,
N-Methyl-L-alanyl-D-lactyl-N-methyl-L-isoleucyl-D-lactyl-N-methyl-L-norleucyl-D-milchsäure,
N-Methyl-L-isoleucyl-D-lactyl-N-methyl-L-norleucyl-D-lactyl-N-methyl-L-alanyl-D-milchsäure,
N-Methyl-L-norleucyl-D-lactyl-N-methyl-L-alanyl-D-lactyl-N-methyl-L-alloisoleucyl-D-milchsäure,
N-Methyl-L-norleucyl-D-lactyl-N-methyl-L-alanyl-D-lactyl-N-methyl-D-isoleucyl-L-milchsäure,
N-Methyl-L-norleucyl-D-lactyl-N-methyl-L-alanyl-D-lactyl-N-methyl-L-isoleucyl-D-milchsäure,
N-Methyl-D-norleucyl-L-lactyl-N-methyl-D-alanyl-D-lactyl-N-methyl-D-isoleucyl-L-milchsäure,
N-Methyl-D-norleucyl-L-lactyl-N-methyl-D-alanyl-L-lactyl-N-methyl-L-isoleucyl-L-milchsäure,
N-Methyl-D-norleucyl-L-lactyl-N-methyl-D-alanyl-L-lactyl-N-methyl-L-isoleucyl-D-milchsäure,
N-Methyl-D-norleucyl-L-lactyl-N-methyl-D-alanyl-L-lactyl-N-methyl-D-isoleucyl-L-milchsäure,
N-Methyl-D-norleucyl-L-lactyl-N-methyl-D-alanyl-L-lactyl-N-methyl-D-alloisoleucyl-L-milchsäure,
N-Methyl-L-leucyl-D-lactyl-N-methyl-D-alanyl-D-lactyl-N-methyl-L-isoleucyl-D-milchsäure,
N-Methyl-L-alanyl-D-lactyl-N-methyl-L-isoleucyl-D-lactyl-N-methyl-L-leucyl-D-milchsäure,
N-Methyl-L-leucyl-D-lactyl-N-methyl-L-norleucyl-D-lactyl-N-methyl-L-alanyl-D-milchsäure,
N-Methyl-L-leucyl-D-lactyl-N-methyl-L-alanyl-D-lactyl-N-methyl-L-alloisoleucyl-D-milchsäure,
N-Methyl-L-leucyl-D-lactyl-N-methyl-L-alanyl-D-lactyl-N-methyl-D-isoleucyl-L-milchsäure,
N-Methyl-L-leucyl-D-lactyl-N-methyl-L-alanyl-D-lactyl-N-methyl-L-isoleucyl-D-milchsäure,
N-Methyl-D-leucyl-L-lactyl-N-methyl-D-alanyl-D-lactyl-N-methyl-D-isoleucyl-L-milchsäure,
N-Methyl-D-leucyl-L-lactyl-N-methyl-D-alanyl-L-lactyl-N-methyl-L-isoleucyl-L-milchsäure,
N-Methyl-D-leucyl-L-lactyl-N-methyl-D-alanyl-L-lactyl-N-methyl-L-isoleucyl-D-milchsäure,
N-Methyl-D-leucyl-L-lactyl-N-methyl-D-alanyl-L-lactyl-N-methyl-D-isoleucyl-L-milchsäure,
N-Methyl-D-leucyl-L-lactyl-N-methyl-D-alanyl-L-lactyl-N-methyl-D-alloisoleucyl-L-milchsäure,
N-Methyl-L-alanyl-D-lactyl-N-methyl-D-isoleucyl-D-lactyl-N-methyl-L-isoleucyl-D-milchsäure,
N-Methyl-L-alloisoleucyl-D-lactyl-N-methyl-L-alanyl-D-lactyl-N-methyl-L-isoleucyl-D-milchsäure,
N-Methyl-L-alanyl-D-lactyl-N-methyl-L-isoleucyl-D-lactyl-N-methyl-L-alloisoleucyl-D-milchsäure,
N-Methyl-L-alanyl-D-lactyl-N-methyl-L-alloisoleucyl-D-lactyl-N-methyl-L-alloisoleucyl-D-milchsäure,
N-Methyl-L-alanyl-D-lactyl-N-methyl-L-isoleucyl-L-lactyl-N-methyl-L-isoleucyl-D-milchsäure,
N-Methyl-L-alanyl-D-lactyl-N-methyl-D-isoleucyl-L-lactyl-N-methyl-L-isoleucyl-D-milchsäure,
N-Methyl-D-alanyl-L-lactyl-N-methyl-D-isoleucyl-D-lactyl-N-methyl-D-isoleucyl-L-milchsäure,
N-Methyl-D-alanyl-L-lactyl-N-methyl-L-isoleucyl-L-lactyl-N-methyl-D-isoleucyl-L-milchsäure,
N-Methyl-D-alanyl-L-lactyl-N-methyl-L-isoleucyl-D-lactyl-N-methyl-D-isoleucyl-L-milchsäure,
N-Methyl-D-alanyl-L-lactyl-N-methyl-D-isoleucyl-L-lactyl-N-methyl-D-isoleucyl-L-milchsäure,
N-Methyl-D-alanyl-L-lactyl-N-methyl-D-alloisoleucyl-L-lactyl-N-methyl-D-isoleucyl-L-milchsäure,
N-Methyl-D-alanyl-L-lactyl-N-methyl-D-isoleucyl-L-lactyl-N-methyl-D-alloisoleucyl-L-milchsäure,
N-Methyl-D-alanyl-L-lactyl-N-methyl-D-alloisoleucyl-L-lactyl-N-methyl-D-alloisoleucyl-L-milchsäure.

Die Cyclisierung der Verbindungen der Formel (II) wird vorzugsweise in Gegenwart geeigneter Kupplungsreagenzien und in Gegenwart eines basischen Reaktionshilfsmittels in einem Verdünnungsmittel unter Hochverdünnungsbedingungen durchgeführt.

Als Kupplungsreagenzien zur Durchführung des Verfahrens 2 finden alle, die zur Herstellung einer Amidbindung geeignet sind (vgl. z.B.: Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis synthesis, biology (Academic Press, New York 1979), Verwendung. Vorzugsweise werden folgende Methoden herangezogen: Aktivestermethode mit Pentachlor- (Pcp) und Pentafluorphenol (Pfp), N-Hydroxysuccinimid, N-Hydroxy-5-norbornen-2,3-dicarboxamid (HONB), 1-Hydroxybenzotriazol (HOBt) oder 3-Hydroxy-4-oxo-3,4-dihydro-1,2,3-benzotriazin als Alkoholkomponente, Kupplung mit Carbodiimiden wie Dicyclohexyl-carbodiimid (DCC) nach dem DCC-Additiv-Verfahren, oder mit n-Propanphosphonsäureanhydrid (PPA) und Gemischt-Anhydrid-Methode mit Pivaloylchlorid, Ethyl- (EEDQ) und Isobutyl-chlorformiat (IIDQ) oder Kupplung mit Phosphoniumreagenzien, wie Benzotriazol-1-yl-oxy-tris(dimethylamino-phosphonium)-hexafluorophosphat (BOP), Bis(2-oxo-3-oxazolidinyl)phosphoniumsäurechlorid (BOP-Cl), oder mit Phosphonsäureesterreagenzien, wie Cyanphosphonsäurediethylester (DEPC) und Diphenylphosphorylazid (DPPA) oder Uroniumreagenzien, wie 2-(1H-Benzotriazol-1-yl)-1,1,3,3-tetra-methyluronium-tetrafluoro-borat (TBTU).

Bevorzugt ist die Kupplung mit Phosphoniumreagenzien wie Bis(2-oxo-3-oxazolidinyl)-phosphoniumsäurechlorid (BOP-Cl), Benzotriazol-1-yl-oxy-tris(dimethylaminophosphonium)-hexafluorophosphat (BOP) und Phosphonsäureesterreagenzien, wie Cyanphosphonsäurediethylester (DEPC) oder Diphenylphosphorylazid (DPPA).

Als basische Reaktionshilfsmittel können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Triethylamin, Trimethylamin, Tribenzylamin, Triisopropylamin, Tributylamin, Tribenzylamin, Tricyclohexylamin, Triamylamin, Trihexylamin, N,N-Dimethyl-anilin, N,N-Di-methyl-toluidin, N,N-Dimethyl-p-aminopyridin, N-Methyl-pyrrolidin, N-Methyl-piperidin, N-Methyl-imidazol, N-Methyl-pyrrol, N-Methyl-morpholin, N-Methyl-hexamethylenimin, Pyridin, 4-Pyrrolidino-pyridin, 4-Dimethylamino-pyridin, Chinolin, α-Picolin, β-Picolin, Isochinolin, Pyrimidin, Acridin, N,N,N',N'-Tetramethylen-diamin, N,N',N'-Tetra-ethylendiamin, Chinoxalin, N-Propyl-diisopropylamin, N-Ethyl-diisopropylamin, N,N'-Dimethyl-cyclohexylamin, 2,6-Lutidin, 2,4-Lutidin, Triethylendiamin, Diazabicyclooctan (DABCO), Diazabicyclononen (DBN) oder Diazabicycloundecen (DBU).

Vorzugsweise finden tertiären Amine, insbesondere Trialkylamine wie Triethylamin, N,N-Diisopropylethylamin oder N-Methylmorpholin, Verwendung.

Als Verdünnungsmittel zur Durchführung des erfindungsgemäßen Verfahrens 2 kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Di-n-butylether, Di-isobutylether, Diisoamylether, Diisopropylether, Anisol, Phenetol, Cyclohexylmethylether, Diethylether, Ethylenglycoldimethylether, Tetrahydrofuran, Dioxan, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Hexan, Nonan, Cymol, Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylformamid, N,N-Dimethylformamid, N-Methyl-pyrrolidon; Ketone wie Aceton, Methylethylketon. Auch Gemische der genannten Lösungs-und Verdünnungsmittel kommen in Betracht.

Bevorzugt sind Halogenkohlenwasserstoffe, insbesondere Chlorkohlenwasserstoffe, wie Methylenchlorid und Chloroform oder Ether, wie beispielsweise Dioxan, und Gemische aus Alkoholen und Ether.

Die als Ausgangsverbindungen verwendeten Hexadepsipeptide können nach klassischen Verfahren hergestellt werden, beispielsweise demjenigen, wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B. F. Gisin (Helv. Chim. Acta 56 (1973) S. 1476) beschrieben ist.

Das Verfahren wird durchgeführt, indem Verbindungen der Formel (II) in Gegenwart eines der angegebenen Kupplungsreagenzien und in Gegenwart eines basischen Reaktionshilfsmittels in einem Verdünnungsmittel unter Hochverdünnungsbedingungen zusammengegeben und gerührt werden. Die Reaktionsdauer beträgt 4 bis 72 Stunden. Die Umsetzung erfogt bei Temperaturen zwischen -5°C und +100°C, bevorzugt zwischen -5°C und +50°C, besonders bevorzugt bei 0°C bis Raumtemperatur. Es wird unter Normaldruck gearbeitet.

Zur Durchführung des erfindungsgemäßen Verfahrens 2 setzt man pro Mol an N-Methyl-L-alanyl-D-lactyl-N-methyl-L-isoleucyl-D-lactyl-N-methyl-L-isoleucyl-D-milchsäure der Formel (II) im allgemeinen 1,0 bis 3,0 Mol, vorzugsweise 1,0 bis 1,5 Mol Kupplungsreagenz, ein.

Nach vollendeter Umsetzung wird die Reaktionslösung schwach alkalisch gewaschen, die organische Phase abgetrennt, getrocknet und im Vakuum eingegengt. Die anfallenden Produkte lassen sich in üblicher Weise durch Umkristallisieren oder Säulenchromatographie reinigen (vgl. auch die Herstellungsbeispiele).

Mit dem Verfahren 2 sind aus offenkettigen Hexadepsipeptiden mit sowohl L-als auch D-Konfiguration aufgebauter Depsipeptidsequenz, Cyclohexadepsipeptide unter Beibehaltung der ursprünglichen Konfiguration der Ausgangsstoffe erhältlich.

Alternativ lassen sich auch die erfindungsgemäßen Verbindungen der Formel (I) nach dem von U. Schmidt et al. für makrocyclische Peptidalkaloide angewendeten Verfahren (vgl. z.B.: U. Schmidt et al. in Synthesis (1991) S. 294-300 [Didemnin A, B und C]; Angew. Chem. 96 (1984) S. 723-724 [Dolastatin 3]; Angew. Chem. 102 (1990) S. 562-563 [Fenestin A]; Angew. Chem. 97 (1985) S. 606-607 [Ulicyclamid]; J. Org. Chem. 47 (1982) S. 3261-3264) darstellen.

Die als Ausgangsmaterialien verwendeten N-Methyl-aminosäuren und 2-Halogencarbonsäurederivate sind teilweise bekannt (vgl. z.B.: N-Methyl-aminosäuren: R. Bowmann et al. J. Chem. Soc. (1950) S. 1346; J. R. McDermott et al. Can. J. Chem. 51 (1973) S. 1915;
H. Wurziger et al., Kontakte (Merck.Darmstadt) 3 (1987) S. 8; 2-Halogencarbonsäurederivate: S. M. Birnbaum et al. J. Amer. Chem. Soc. 76 (1954) S. 6054, C. S. Rondestvedt, Jr. et al. Org. Reactions 11 (1960) S. 189 [Review]) bzw. können nach den dort beschriebenen Verfahren erhalten werden.

Für die Kupplungsreaktion zur Darstellung der als Ausgangsverbindungen eingesetzten Depsipeptide (III), (IV), (V), (VI) und (VII) finden alle Kupplungsreagenzien, die zur Herstellung einer Amidbindung geeignet sind (vgl. z.B.: Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis synthesis, biology (Academic Press, New York 1979), Verwendung.

Die offenkettigen Hexadepsipeptide (II) können beispielsweise für das [L-D-L-D-L-D]-Isomer nach einer Reaktionssequenz erhalten werden werden, die die nachfolgenden Stufen umfaßt:
a) Synthese der [L-D]-konfigurierten Didepsipeptide der Formeln (V) bis (VII): 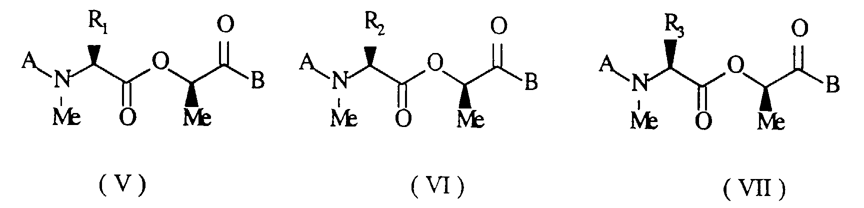 worin A eine N-terminale Schutzgruppe, wie z.B. die Benzyl oder Benzyloxycarbonylgruppe und B eine C-terminale Schutzgruppe, wie z.B. die tert-Butoxygruppe, darstellt.
   Dies entspricht beispielsweise für Formel (VIIa),
   worin B für tert.-Butyloxy steht, dem folgenden Reaktionsschema: 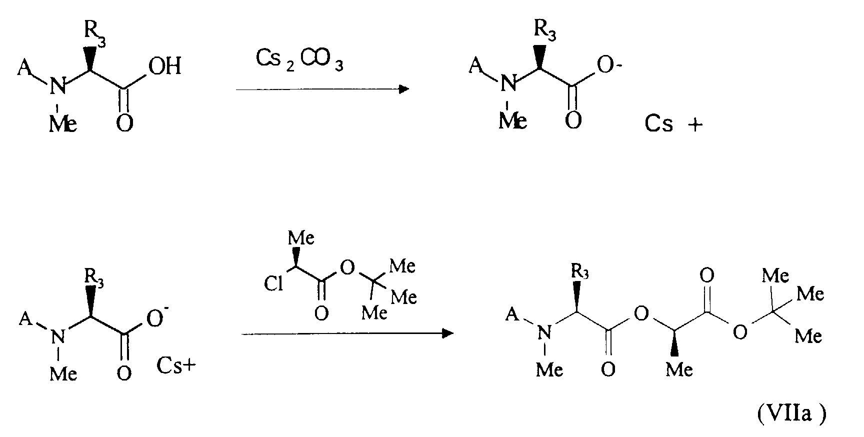 Die Herstellung der enantiomerenreinen Verbindungen der Formeln (V), (VI) und (VII) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.
   Am Ende dieser Stufe kann entweder eine Entfernung der N-terminalen Schutzgruppe aus den Derivaten der Formel (VIIa) in an sich bekannter Weise durchgeführt werden, beispielsweise durch katalytische Hydrierung, zur Herstellung der Derivate der Formel (VIIc) oder es kann in an sich bekannter Weise eine Abspaltung der C-terminalen Schutzgruppe aus den Derivaten der Formel (V) und (VI), vorzugsweise durch Acidolyse, zur Darstellung der Derivate (Vb) und (VIb) erfolgen: 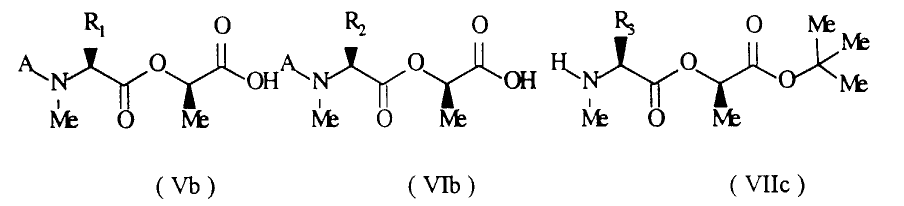
b) Synthese der [L-D-L-D]-konfigurierten Tetradepsipeptide der Formel (III) und (IV) 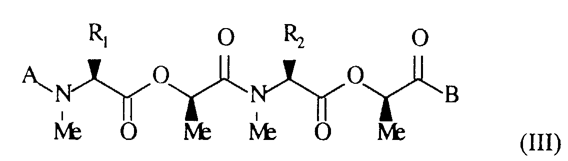 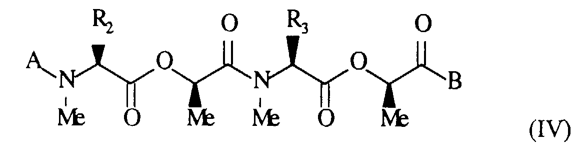 dies entspricht beispielsweise für Formel (IVa),
   worin B für tert.-Butyloxy steht, dem nachfolgenden Reaktionsschema: 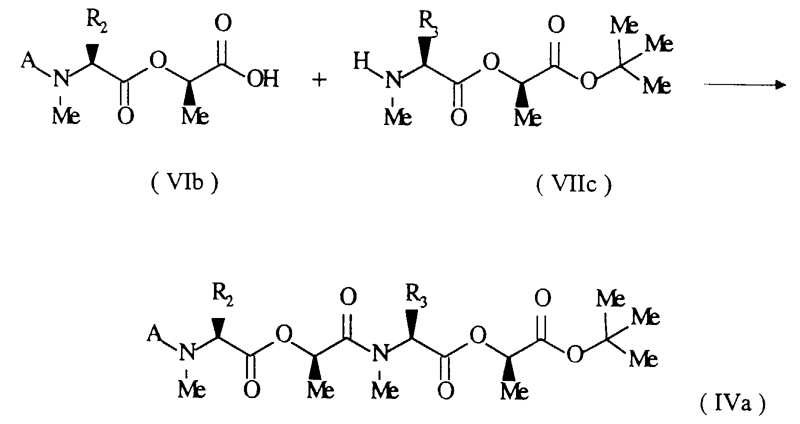 Man kann anschließend eine Entfernung der N-terminalen Schutzgruppe aus den Derivaten der Formel (IVa) durchführen, beispielsweise durch katalytische Hydrierung wie oben angegeben, zur Herstellung der Derivate der Formel 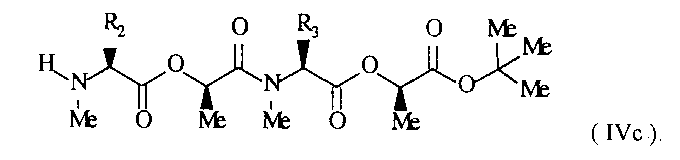
c) Synthese der offenkettigen [L-D-L-D-L-D]-konfigurierten Hexadepsipeptide der Formel 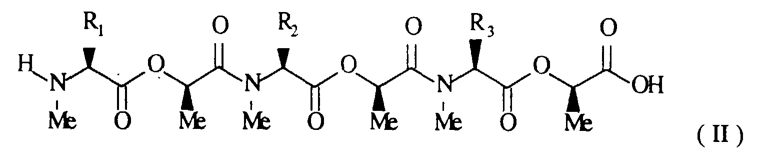 nach der folgenden Reaktionsgleichung: 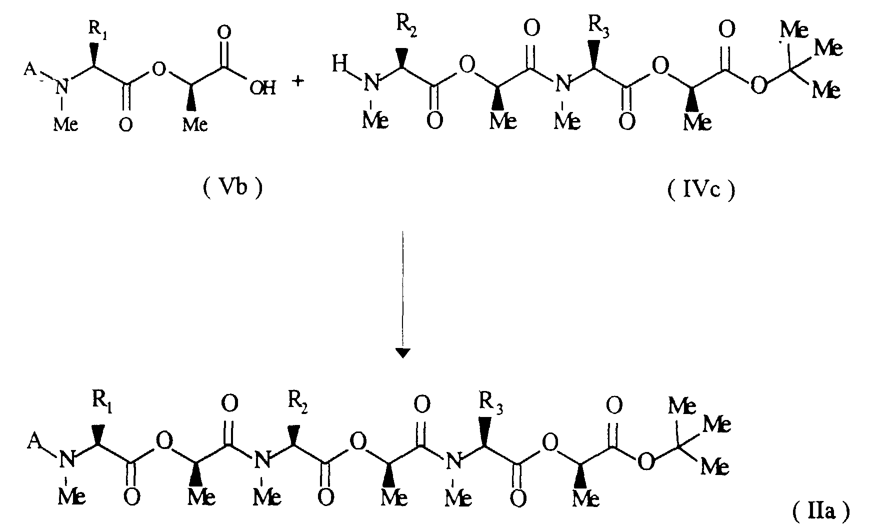 Man kann anschließend eine Entfernung der C-terminalen Schutzgruppe aus den Derivaten der Formel (IIa) in an sich bekannter Weise, beispielsweise durch Acidolyse, zur Herstellung der Derivate 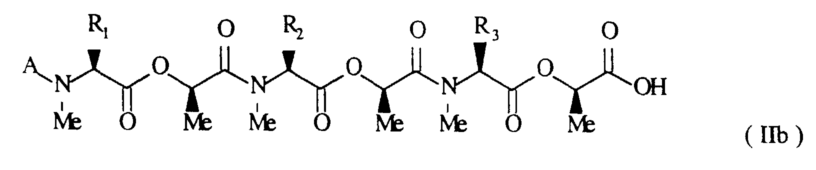 durchführen oder man deblockiert die Derivate der Formel (IIa) N-terminal in an sich bekannter Weise, beispielsweise durch katalytische Hydrierung wie oben angegeben, zur Herstellung der Derivate der Formel 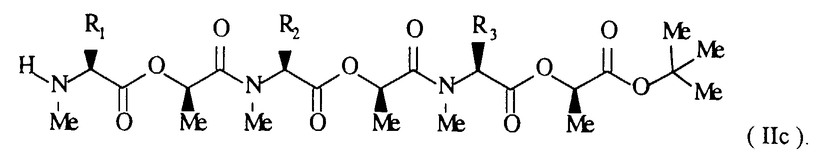 Am Ende dieser Stufen kann eine Entfernung der N-terminalen bzw. C-terminalen Schutzgruppe aus den Derivaten der Formel (IIb) bzw (IIc) in an sich bekannter Weise durchgeführt werden, beispielsweise durch katalytische Hydrierung oder durch Acidolyse wie oben angegeben, zur Herstellung der Derivate der Formel 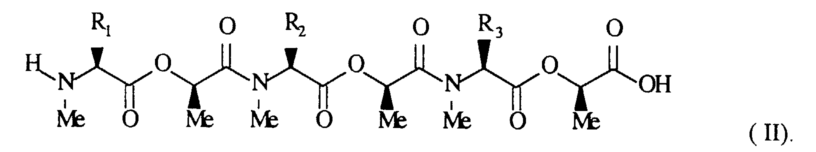 Die anfallenden Produkte lassen sich in üblicher Weise durch Umkristallisieren, oder durch Säulenchromatographie reinigen (vgl. auch die Herstellungsbeispiele).

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp.

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasseifische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprähens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.
Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methyipyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl / Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat; Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureesteronoethynolaminsalz;
Als weitere Hilfsstoffe seien genannt: Viskositätserhöhende und die Emulsion stabilisierende Stoffe wie Carboxymethylcellulose, Methylcellulose und andere Cellulose-und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside ganannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 1 | 10 |
| 2 | 10 |
| 5 | 10 |

### Beispiel B

### In vivo Nematodentest

### Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel-Nr. | Dosis effectiva in mg/kg |
|---|---|
| 1 | 10 |
| 5 | 10 |
| 10 | 10 |
| 16 | 10 |

### Herstellungsbeispiele

### Beispiel 1:

### Cyclo(-N-methy-L-alany-D-lactyl-N-methy-L-isoleucyl-D-lactyl-N-methyl-L-isoleucyl-D-lactyl-)

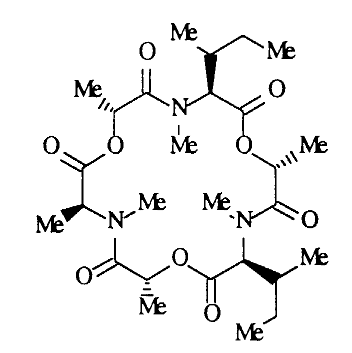

Zu einer Lösung von 0,5 g (0,87 mMol) N-Methyl-L-alanyl-D-lactyl-N-methyl-L-isoleucyl-D-lactyl-N-methyl-L-isoleucyl-D-milchsäure in 500 ml Methylenchlorid werden bei 0°C 0,28 g (2,18 mMol) N,N-Diisopropylethylamin ("Hünig's Base") sowie 0,27 g (1,05 mMol) Bis(2-oxo-3-oxazolidinyl)-phosphoniumsäurechlorid (BOP-Cl) zugegeben und 24 Stunden bei Raumtemperatur gerührt. Anschließend wird nochmals bei 0°C mit 0,28 g (2,18 mMol) N,N-Diisopropylethylamin ("Hünig's Base") sowie 0,27 g (1,05 mMol) Bis(2-oxo-3-oxazolidinyl)-phosphoniumsäurechlorid (BOP-Cl) versetzt und weitere 24 Stunden bei Raumtemperatur gerührt. Die Reaktionslösung wird zweimal mit Wasser gewaschen, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0.04 bis 0,063 mm) mit dem Fließmittel Toluol: Essigsäureethylester (2:1) chromatographiert. Man erhält 0.29 g (59,8 % der Theorie) Cyclo(-N-methyl-L-alanyl-D-lactyl-N-methyl-L-isoleucyl-D-lactyl-N-methyl-L-isoleucyl-D-lactyl-).

¹³C-NMR (100 MHz, CDCl₃, δ): 10,6; 10,7; 13,5; 15,4; 15,9; 16,0; 16,8; 17,1 (-CH₃); 24,4; 24,5 (-CH₂-); 30,9; 31,5; (-CH-); 32,8; 33,9; 34,2 (-N-CH₃); 56,2; 59,9; 60,5 (-N-CH-); 66,0; 66,3; 67,7 (-O-CH-); 168,7; 169,7; 170,1 (-CO-N-); 169,0; 170,0; 170,4 (-CO-O-) ppm
EI-MS m/z (%): 555 (M⁺,64); 499 (37); 483 (34); 428 (12); 357 (19); 340 (5); 255 (50); 182 (100); 100 (52)
Analog können die in den nachstehenden Tabellen 1 bis 4 aufgeführten Verbindungen der Formel (I) hergestellt werden.

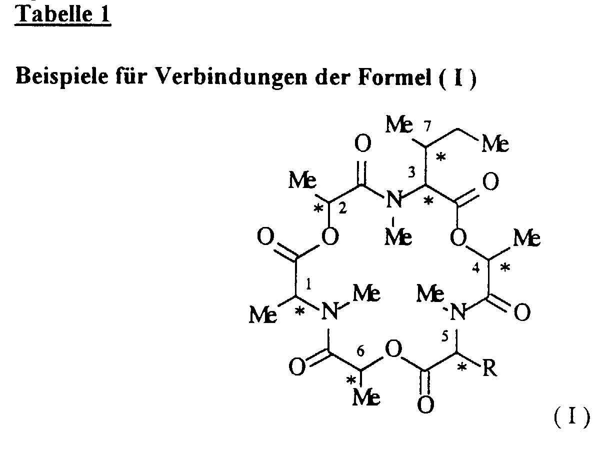

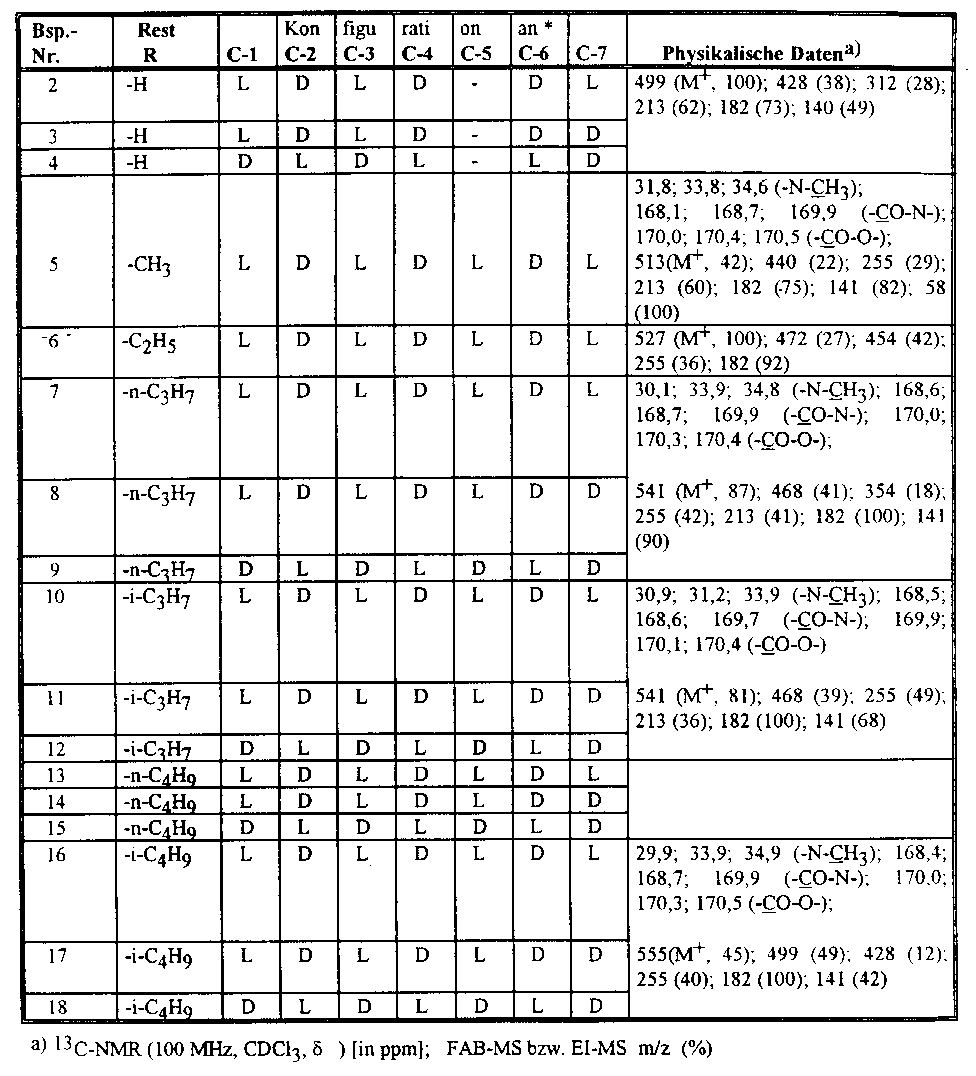

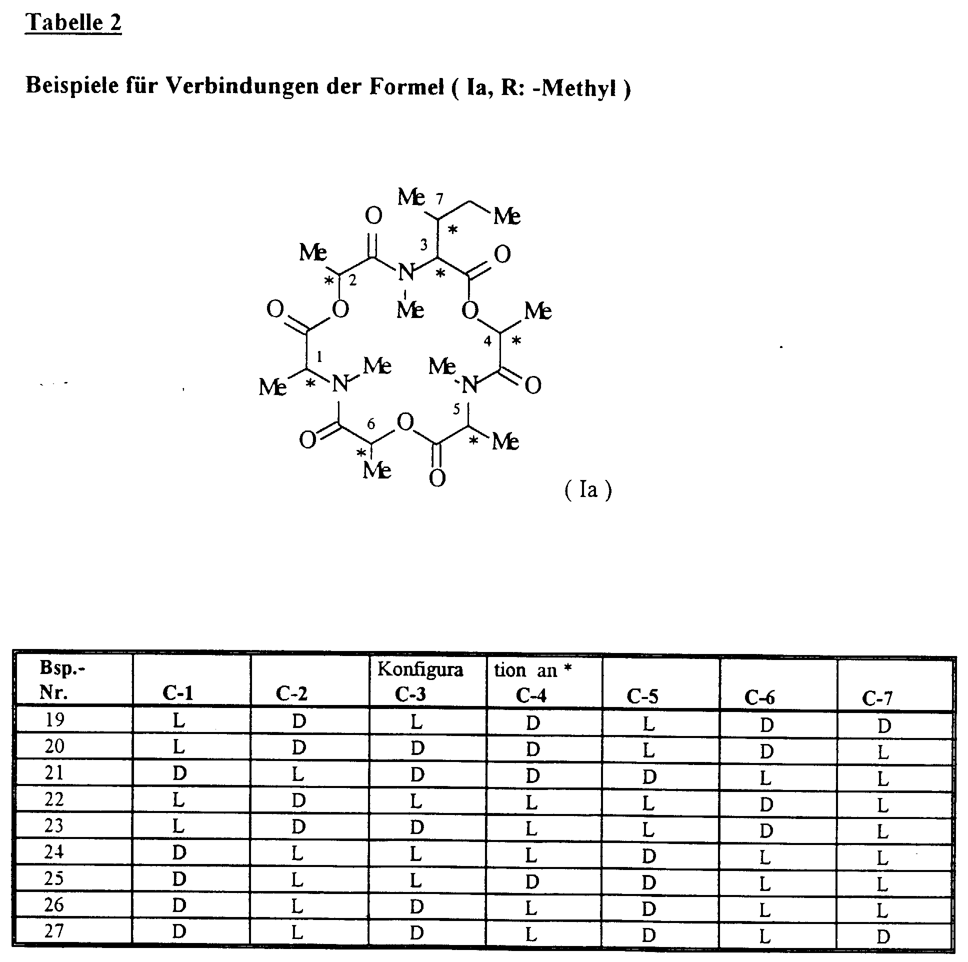

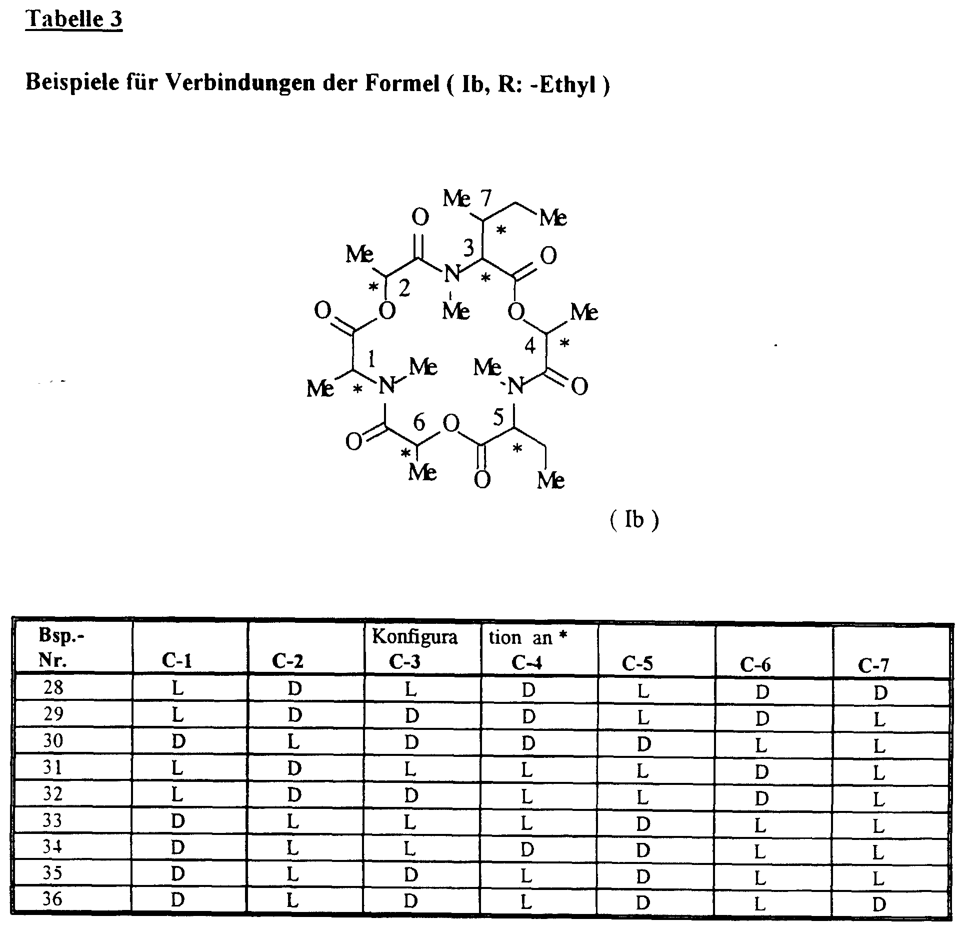

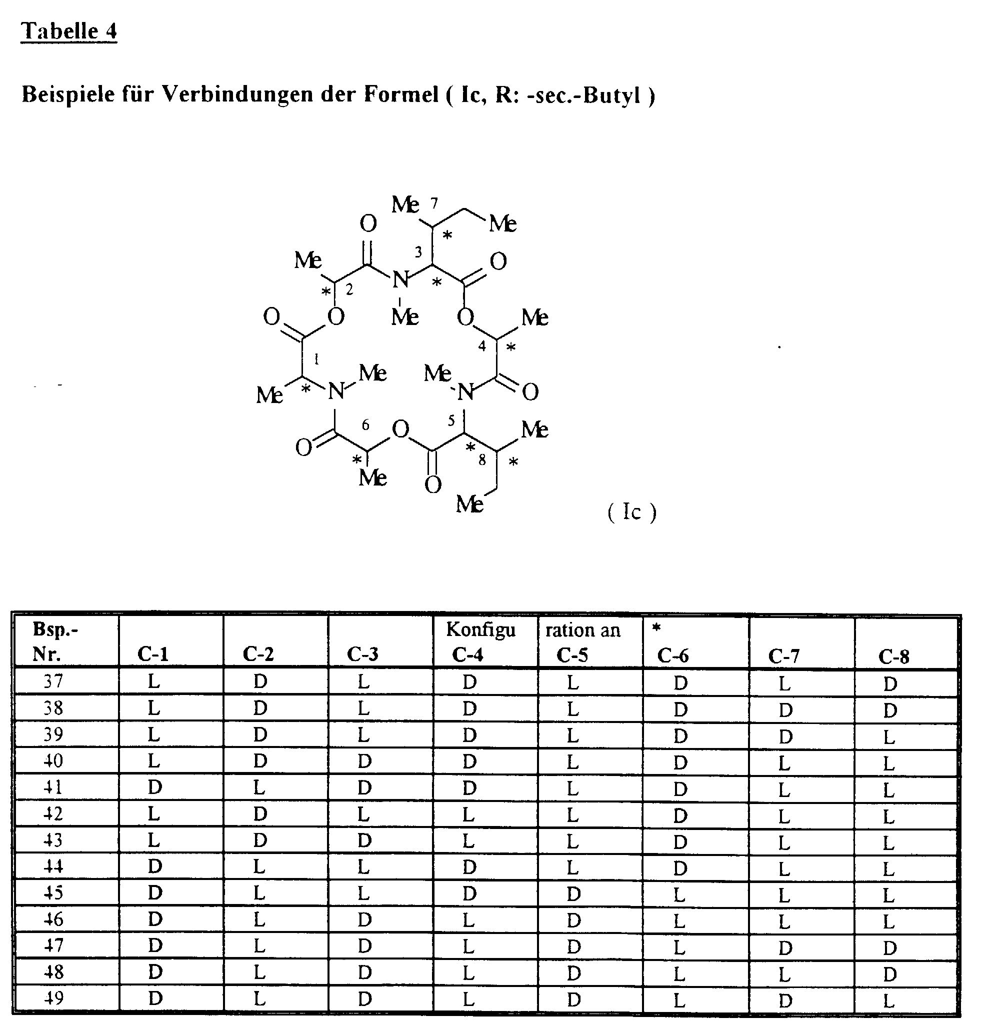

### Ausgangsstoffe der Formel (II)

### Beispiel (II-1)

### N-Benzyloxycarbony-N-methyl-L-alanyl-D-lactyl-N-methyl-L-isoleucyl-D-lactyl-N-methyl-L-isoleucyl-D-milchsäure-tert.-butylester

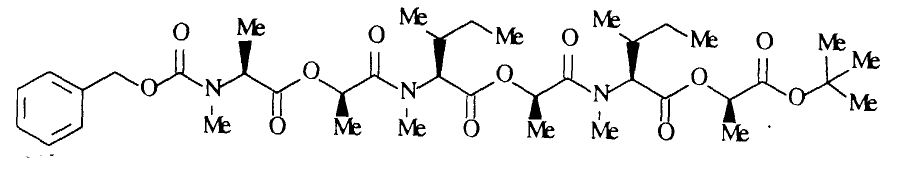

Zu einer Lösung von 5,0 g (16,2 mMol) N-Benzyloxycarbonyl-N-methyl-L-alanyl-D-milchsäure und 7,6 g (16,2 mMol) N-Methyl-L-isoleucyl-D-lactyl-N-methyl-L-isoleucyl-D-milchsäure-tert.-butylester in 200 ml Methylenchlorid werden bei 0°C 4,6 g (35,6 mMol) N,N-Diisopropylethylamin ("Hünig's Base") sowie 4,5 g (17,8 mMol) Bis(2-oxo-3-oxazolidinyl)-phosphoniumsäurechlorid (BOP-Cl) zugegeben und 4 Stunden bei Raumtemperatur gerührt. Die Reaktionslösung wird zweimal mit Wasser gewaschen, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Toluol: Essigsäureethylester (3:1) chromatographiert. Man erhält 7,4 g (59,9 % der Theorie) N-Benzyloxycarbonyl-N-methyl-L-alanyl-D-lactyl-N-methyl-L-isoleucyl-D-lactyl-N-methyl-L-isoleucyl-D-milchsäure-tert.-butylester.

EI-MS m/z (%): 763 (M⁺,10); 707 (M⁺-CH₂=CMe₂, 12); 690 (M⁺-OCMe₃, 7); 651 (9); 491 (19); 419 (34); 386 (24); 292 (25); 183 (41); 148 (42); 91 (Ph-CH₂, 100)

### Beispiel (II-2)

### N-Benzyl-N-methyl-L-alanyl-D-lactyl-N-methyl-L-isoleucyl-D-lactyl-N-methyl-L-isoleucyl-D-milchsäure-tert.-butylester

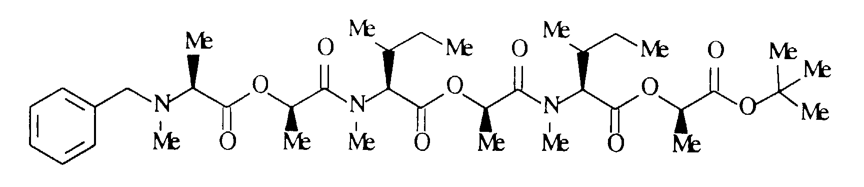

Die Kupplungsreaktion erfolgt analog der Reaktionsvorschrift des Beispiels (II-1) unter Verwendung von:
14,0 g (52,9 mMol) N-Benzyl-N-methyl-L-alanyl-D-milchsäure,
25,0 g (52,9 mMol) N-Methyl-L-isoleucyl-D-lactyl-N-methyl-L-isoleucyl-D-milch säure-tert.-butylester,
500 ml Methylenchlorid,
15,0 g (116,3 mMol) N,N-Diisopropylethylamin ("Hünig's Base"),
14,8 g (58,2 mMol) Bis(2-oxo-3-oxazolidinyl)-phosphoniumsäurechlorid (BOP-Cl).

Das zurückbleibende Rohprodukt wird über eine Kieselgelsäule (Kieselgel 60 - Merck, Korngröße: 0,04 bis 0,063 mm) mit dem Fließmittel Cyclohexan : Essigsäureethylester (1:1) chromatographiert. Man erhält 22,0 g (57,8 % der Theorie) N-Benzyl-N-methyl-L-alanyl-D-lactyl-N-methyl-L-isoleucyl-D-lactyl-N-methyl-L-isoleucyl-D-milchsäure-tert.-butylester.

EI-MS m/z (%): 719 (M⁺,45); 646 (50); 600 (80); 544 (82); 148 (100)

### Beispiel (II-3)

### N-Benzyloxycarbonyl-N-methyl-L-alanyl-D-lactyl-N-methyl-L-isoleucyl-D-lactyl-N-methyl-L-isoleucyl-D-milchsäure

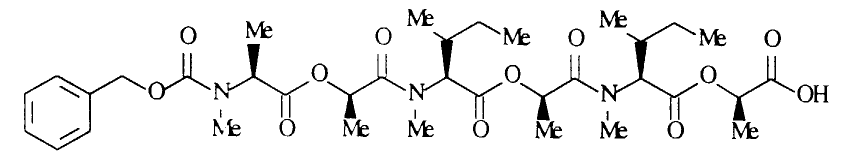

In eine auf 0°C gekühlte Lösung von 6,2 g (8,12 mMol) N-Benzyloxycarbonyl-N-methyl-L-alanyl-D-lactyl-N-methyl-L-isoleucyl-D-lactyl-N-methyl-L-isoleucyl-D-milchsäure-tert.-butylester in 220 ml absolutem Methylenchlorid wird 20 Minuten trockenes Chlorwasserstoffgas eingeleitet. Anschließend rührt man ca. 16 Stunden bei Raumtemperatur und engt den gesamten Reaktionsansatz im Vakuum ein. Man erhält 4,9 g (85,3 % der Theorie) N-Benzyloxycarbonyl-N-methyl-L-alanyl-D-lactyl-N-methyl-L-isoleucyl-D-lactyl-N-methyl-L-isoleucyl-D-milchsäure, die ohne weitere Reinigung weiter umgesetzt werden kann.

MS m/z (%): 708 (M⁺+H, 1); 707 (M⁺, 6); 651 (3); 572 (1); 436 (3); 292 (3); 192 (8); 148 (30); 91 (Ph-CH₂, 100)

### Beispiel (II-4)

### N-Benzyl-N-methyl-L-alanyl-D-lactyl-N-methyl-L-isoleucyl-D-lactyl-N-methyl-L-isoleucyl-D-milchsäure

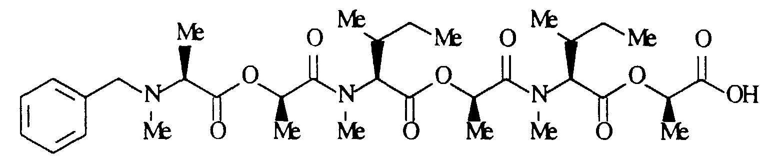

Die C-terminale Acidolyse erfolgt analog der Reaktionsvorschrift des Beispiels (II-3) unter Verwendung von:
21,8 g (30,3 mMol) N-Benzyl-N-methyl-L-alanyl-D-lactyl-N-methyl-L-isoleucyl-D-lactyl-N-methyl-L-isoleucyl-D-milchsäure-tert.-butylester,
500 ml Methylenchlorid.

Man erhält 20,0 g (99,5 % der Theorie) N-Benzyl-N-methyl-L-alanyl-D-lactyl-N-methyl-L-isoleucyl-D-lactyl-N-methyl-L-isoleucyl-D-milchsäure, die ohne weitere Reinigung weiter umgesetzt werden kann.

FAB-MS m/z (%): 665 (M⁺+H, 37); 664 (M⁺,100); 148 (82)

### Beispiel (II-5)

### N-Methyl-L-alanyl-D-lactyl-N-methyl-L-isoleucyl-D-lactyl-N-methyl-L-isoleucyl-D-milchsäure

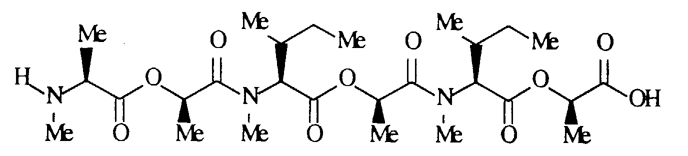

4,5 g (6,36 mMol) N-Benzyloxycarbonyl-N-methyl-L-alanyl-D-lactyl-N-methyl-L-isoleucyl-D-lactyl-N-methyl-L-isoleucyl-D-milchsäure werden in 180 ml Ethanol in Gegenwart von 0,45 g Pd(OH)₂/Kohle [20 % Pd-Gehalt] bis zur Beendigung der Wasserstoffaufnahme hydriert (ca. 4 Stunden). Nach Abfiltrieren des Katalysators wird die gesamte Reaktionslösung im Vakuum eingeengt.

Man erhält 3,6 g (98,7 % der Theorie) N-Methyl-L-alanyl-D-lactyl-N-methyl-L-isoleucyl-D-lactyl-N-methyl-L-isoleucyl-D-milchsäure, die ohne weitere Reinigung cyclisiert werden kann.

EI-MS m/z (%): 573 (M⁺, 2); 472 (14), 386 (34); 285 (17); 183 (82); 155 (27); 100 (60); 58 (MeNH-CHMe, 100)
Die Darstellung von:

### Beispiel (II-6)

### N-Methyl-L-isoleucyI-D-lactyl-N-methyl-L-alanyl-D-lactyl-N-methyl-L-isoleucyl-D-milchsäure

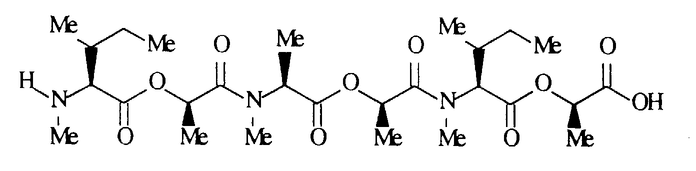

und

### Beispiel (II-7)

### N-Methyl-L-isoleucyl-D-lactyl-N-methyl-L-isoleucyl-D-lactyl-N-methyl-L-alanyl-D-milchsäure

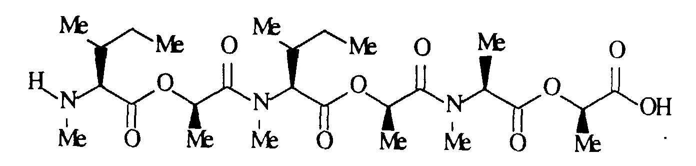

erfolgt analog der Reaktionsvorschrift des Beispiels (II-5)
Analog können die in der nachstehenden Tabelle 5 aufgeführten Ausgangsstoffe der allgemeinen Formel (II) als [L-D-L-D-L-D]-Stereoisomere hergestellt werden:

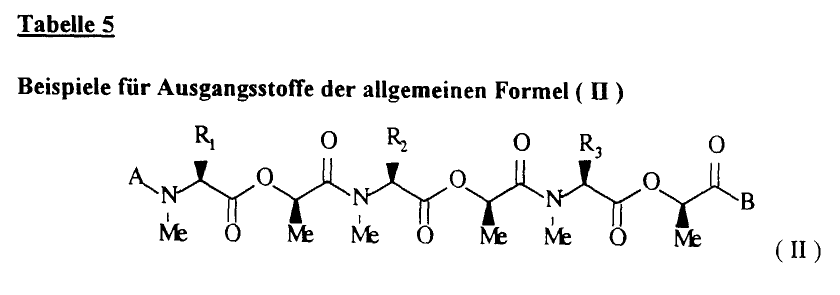

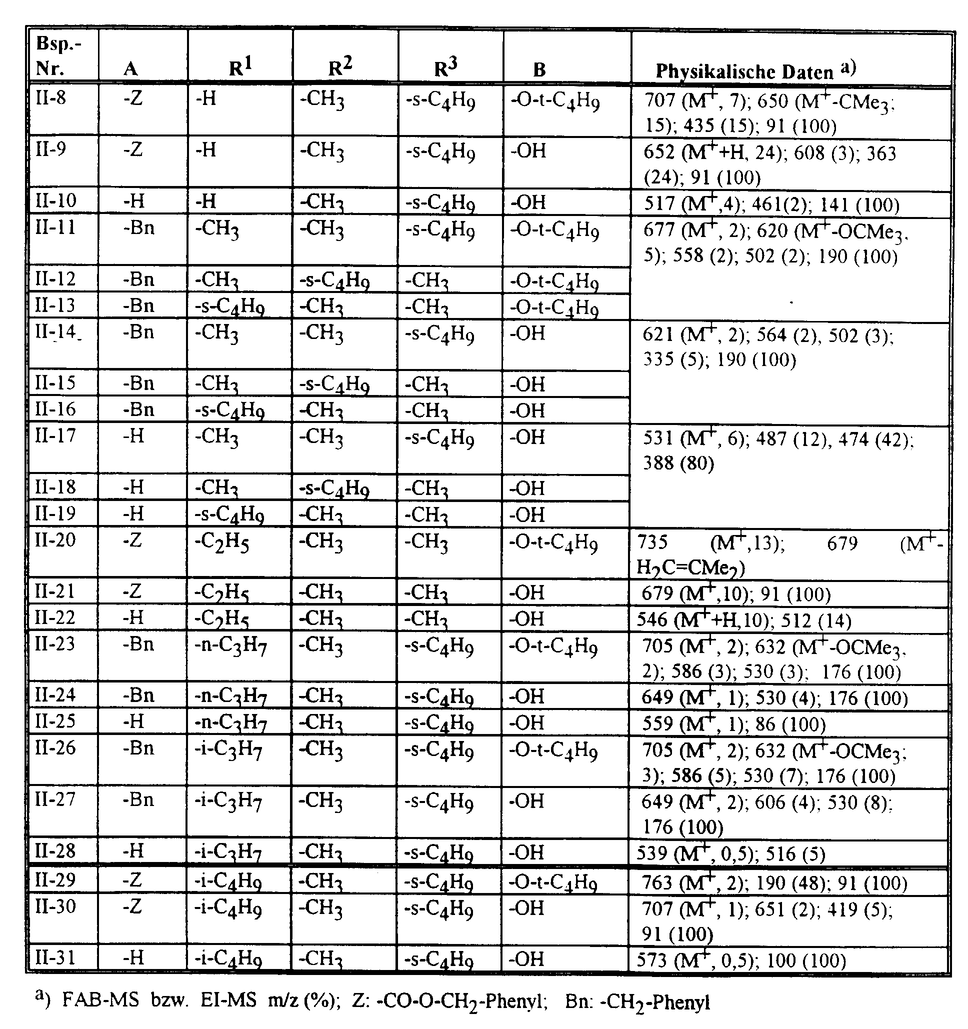

### Ausgangsstoffe der Formel (III)

### Beispiel (III-1)

### N-Benzyl-N-methyl-L-isoleucyl-D-lactyl-N-methyl-L-isoleucyl-D-milchsäure -tert.-butylester

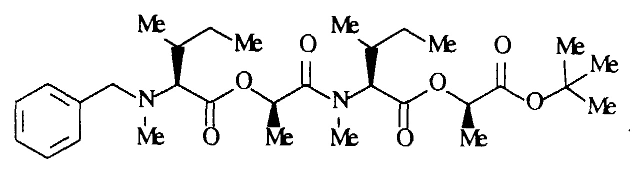

Die Kupplungsreaktion erfolgt analog der Reaktionsvorschrift des Beispiels (II-1) unter Verwendung von:
20,4 g (66,6 mMol) N-Benzyl-N-methyl-L-isoleucyl-D-milchsäure,
18,2 g (66,6 mMol) N-Methyl-L-isoleucyl-D-milchsäure-tert.-butylester,
500 ml Methylenchlorid,
18,9 g (146,4 mMol) N,N-Diisopropylethylamin ("Hünig's Base"),
18,6 g (73,2 mMol) Bis(2-oxo-3-oxazolidinyl)-phosphoniumsäurechiorid (BOP-Cl).

Man erhält 37,0 g (98,8 % der Theorie) N-Benzyl-N-methyl-L-isoleucyl-D-lactyl-N-methyl-L-isoleucyl-D-milchsäure-tert.-butylester der ohne weitere Reinigung weiter umgesetzt werden kann.

EI-MS m/z (%): 563 (M⁺+H, 1); 562 (M⁺, 3); 505 (M⁺-CMe₃, 7); 489 (M⁺-OCMe₃, 6); 190 (100)

### Beispiel (III-2)

### N-Benzyl-N-methyl-L-alanyl-D-lactyl-N-methyl-L-isoleucyl-D-milchsäure -tert.-butylester

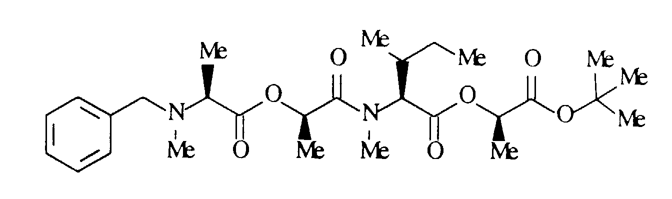

Die Kupplungsreaktion erfolgt analog der Reaktionsvorschrift des Beispiels (II-1) unter Verwendung von:
13,6 g (51,3 mMol) N-Benzyl-N-methyl-L-alanyl-D-milchsäure,
14,0 g (51,3 mMol) N-Methyl-L-isoleucyl-D-milchsäure-tert.-butylester,
300 ml Methylenchlorid,
14,6 g (112,7 mMol) N-N-Diisopropylethylamin ("Hünig's Base"),
14,4 g (56,4 mMol) Bis(2-oxo-3-oxazolidinyl)-phosphoniumsäurechlorid (BOP-Cl).

Man erhält 26,7 g (100 % der Theorie) N-Benzyl-N-methyl-L-alanyl-D-lactyl-N-methyl-L-isoleucyl-D-milchsäure-tert.-butylester der ohne weitere Reinigung weiter umgesetzt werden kann.

EI-MS m/z (%): 520 (M⁺,46); 447 (M⁺ - OCMe₃, 77); 345 (42); 148 (100)

### Beispiel (III-3)

### N-Benzyl-N-methyl-L-isoleucyl-D-lactyl-N-methyl-L-alanyl-D-milchsäure-tert.-butylester

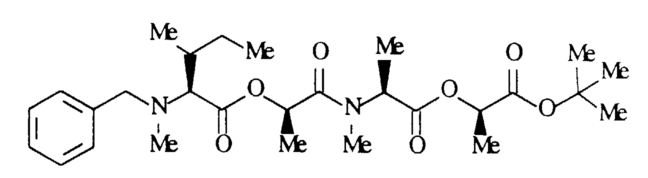

Die Kupplungsreaktion erfolgt analog der Reaktionsvorschrift des Beispiels (II-1) unter Verwendung von:
5,0 g (16,3 mMol) N-Benzyl-N-methyl-L-isoleucyl-D-milchsäure,
3,7 g (16,3 mMol) N-Methyl-L-alanyl-D-milchsäure-tert.-butylester,
150 ml Methylenchlorid,
4,6 g (35,7 mMol) N,N-Diisopropylethylamin ("Hünig's Base"),
4,6 g (17,9 mMol) Bis(2-oxo-3-oxazolidinyl)-phosphoniumsäurechlorid (BOP-Cl).

Das zurückbleibende Rohprodukt wird über eine Kieselgelsäule (Kieselgel 60 - Merck, Korngröße: 0,04 bis 0,063 mm) mit dem Fließmittel Cyclohexan : Essigsäureethylester (6:1) chromatographiert. Man erhält 5,1 g (60,4 % der Theorie) N-Benzyl-N-methyl-L-isoleucyl-D-lactyl-N-methyl-L-alanyl-D-milchsäure-tert.-butylester.

EI-MS m/z (%): 520 (M⁺,58); 463 (M⁺ - CMe₃, 76); 447 (M⁺ - OCMe₃,91); 190 (100)

### Beispiel (III-4)

### N-Benzyl-N-methyl-L-alanyl-D-lactyl-N-methyl-L-alanyl-D-milchsäure-tert.-butylester

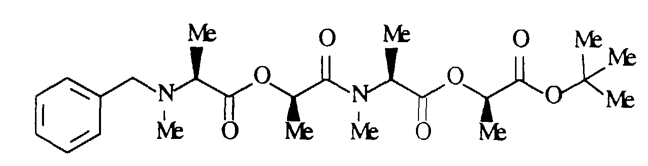

Die Kupplungsreaktion erfolgt analog der Reaktionsvorschrift des Beispiels (II-1) unter Verwendung von:
24,8 g (93,3 mMol) N-Benzyl-N-methyl-L-alanyl-D-milchsäure,
21,4 g (93,3 mMol) N-Methyl-L-alanyl-D-milchsäure-tert.-butylester,
500 ml Methylenchlorid,
26,5 g (205,1 mMol) N,N-Diisopropylethylamin ("Hünig's Base"),
26,1 g (102,6 mMol) Bis(2-oxo-3-oxazolidinyl)-phosphoniumsäurechlorid (BOP-Cl).

Das zurückbleibende Rohprodukt wird über eine Kieselgelsäule (Kieselgel 60 - Merck, Korngröße : 0,04 bis 0,063 mm) mit dem Fließmittel Cyclohexan: Essigsäureethylester (2:1) chromatographiert. Man erhält 16,5 g (36,9 % der Theorie) N-Benzyl-N-methyl-L-alanyl-D-lactyl-N-methyl-L-alanyl-D-milchsäure-tert.-butylester.

EI-MS m/z (%): 478 (M⁺,1); 405 (M⁺ - OCMe₃, 6); 148 (100)

### Beispiel (III-5)

### N-Benzyl-N-methyl-L-isoleucyl-D-lactyl-N-methyl-L-isoleucyl-D-milchsäure-tert.-butylester

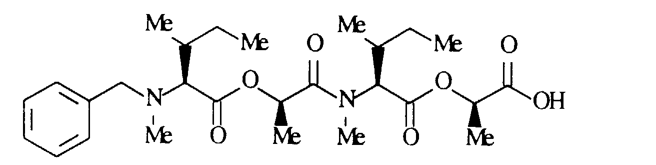

Die C-terminale Acidolyse erfolgt analog der Reaktionsvorschrift des Beispiels (II-3) unter Verwendung von:
9,0 g (15,9 mMol) N-Benzyl-N-methyl-L-isoleucyl-D-lactyl-N-methyl-L-isoleucyl-D-milchsäure-tert.-butylester,
300 ml Methylenchlorid.

Man erhält 8,2 g (100 % der Theorie) N-Benzyl-N-methyl-L-isoleucyl-D-milchsäure, die ohne weitere Reinigung weiter umgesetzt werden kann.

EI-MS m/z (%): 506 (M⁺,2); 449 (8); 190 (100)

### Ausgangsstoffe der Formel (IV)

### Beispiel (IV-1)

### N-Methyl-L-isoleucyl-D-lactyl-N-methyl-L-isoleucyl-D-milchsäure-tert.-butylester

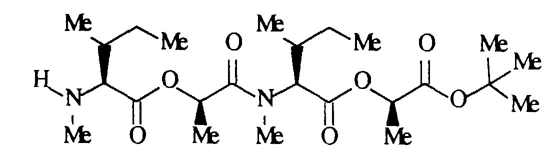

Die N-terminale Deblockierung erfolgt analog der Reaktionsvorschrift des Beispiels (II-5) innerhalb von ca. 4 Stunden unter Verwendung von:
35,0 g (62,2 mMol) N-Benzyloxycarbonyl-N-methyl-L-isoleucinyl-D-lactyl-N-methyl-L-isoleucyl-D-milchsäure-tert.-butylester,
3,5 g Pd(OH)₂/Kohle [20 % Pd-Gehalt],
700 ml Ethanol

Man erhält 25,1 g (85,4 % der Theorie) N-Methyl-L-isoleucyl-D-lactyl-N-methyl-L-isoleucyl-D-milchsäure-tert.-butylester, der ohne weitere Reinigung für die Kupplungsreaktion verwendet werden kann.

EI-MS m/z (%): 472 (M⁺,6); 359 (6); 273 (6); 100 (100)

### Beispiel (IV-2)

### N-Methyl-L-alanyl-D-lactyl-N-methyl-L-isoleueyl-D-milchsäure-tert.-butylester

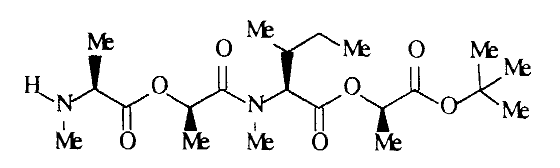

Die N-terminale Deblockierung erfolgt analog der Reaktionsvorschrift des Beispiels (II-5) innerhalb von ca. 4 Stunden unter Verwendung von:
26,3 g (50,5 mMol) N-Benzyl-N-methyl-L-alanyl-D-lactyl-N-methyl-L-isoleucyl-D-milchsäure-tert.-butylester,
3,0 g Pd(OH)₂/Kohle [20 % Pd-Gehalt],
500 ml Ethanol

Man erhält 21,3 g (98,0 % der Theorie) N-Methyl-L-alanyl-D-lactyl-N-methyl-L-isoleucyl-D-milchsäure-tert.-butylester, der ohne weitere Reinigung für die Kupplungsreaktion verwendet werden kann.

### Beispiel (IV-3)

### N-Methyl-L-isoleucyl-D-lactyl-N-methyl-L-alanyl-D-milchsäure-tert.-butylester

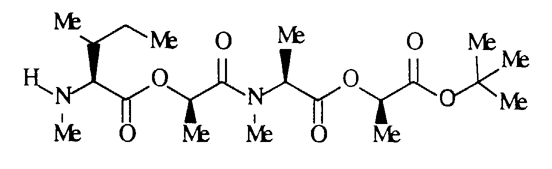

Die N-terminale Deblockierung erfolgt analog der Reaktionsvorschrift des Beispiels (II-5) innerhalb von ca. 4 Stunden unter Verwendung von:
4,8 g (9,3 mMol) N-Benzyl-N-methyl-L-isoleucyl-D-lactyl-N-methyl-L-alanyl-D-milchsäure-tert.-butylester,
0,5 g Pd(OH)₂/Kohle [20 % Pd-Gehalt],
100 ml Ethanol

Man erhält 3,2 g (83,2 % der Theorie) N-Methyl-L-isoleucyl-D-lactyl-N-methyl-L-alanyl-D-milchsäure-tert.-butylester, der ohne weitere Reinigung für die Kupplungsreaktion verwendet werden kann.

FAB-MS m/z (%): 431 (M⁺+H, 12); 190 (100)

### Beispiel (IV-4)

### N-Methyl-L-alanyl-D-lactyl-N-methyl-L-alanyl-D-milchsäure-tert.-butylester

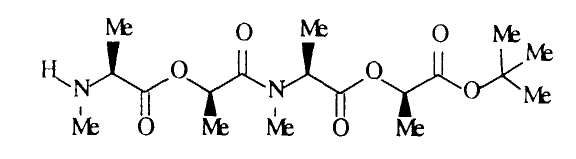

Die N-terminale Deblockierung erfolgt analog der Reaktionsvorschrift des Beispiels (II-5) innerhalb von ca. 4 Stunden unter Verwendung von:
15,0 g (32,0 mMol) N-Benzyl-N-methyl-L-alanyl-D-lactyl-N-methyl-L-alanyl-D-milchsäure-tert.-butylester,
1,5 g Pd(OH)₂/Kohle [20 % Pd-Gehalt],
400 ml Ethanol

Man erhält 10,8 g (89,5 % der Theorie) N-Methyl-L-alanyl-D-lactyl-N-methyl-L-alanyl-D-milchsäure-tert.-butylester, der ohne weitere Reinigung für die Kupplungsreaktion verwendet werden kann.

FAB-MS m/z (%): 388 (M⁺, 0,5); 344 (M⁺-CO₂, 4); 315 (15); 58 (100)

### Ausgangsstoffe der Formel (V), (VI) und (VII)

### Beispiel (V-1)

### N-Benzyl-N-methyl-L-alanyl-D-milchsäure-tert.-butylester

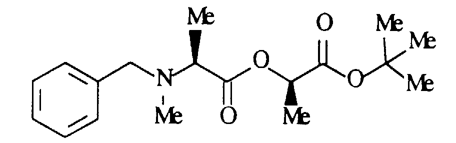

7,0 g (36,2 mMol) N-Benzyl-N-methyl-L-alanin werden in 155 ml Methanol und 15 ml Wasser gelöst, mit 30 ml einer 20 %igen Cäsiumcarbonat-Lösung versetzt und ca. eine Stunde bei Raumtemperatur gerührt. Anschließend wird dreimal mit ca. 100 ml absolutem Dimethylformamid versetzt, im Vakuum eingeengt und im Hochvakuum getrocknet. Das Cäsiumsalz wird in 76 ml Dimethylformamid vorgelegt, mit 6,0 g (36,2 mMol) L-2-Chlor-milchsäure-tert.-butylester versetzt und ca. 18 Stunden bei Raumtemperatur gerührt.
Die gesamte Reaktionslösung wird im Vakuum eingeengt, der ölige Rückstand in Methylenchlorid aufgenommen und zweimal mit Wasser geschüttelt. Danach wird die organische Phase abgetrennt, über Natriumsulfat getrocknet und im Vakuum eingeengt.

Man erhält 8,9 g (76,5 % der Theorie) N-Benzyl-N-methyl-L-alanyl-D-milchsäure-tert.-butylester, der ohne Reinigung weiter umgesetzt werden kann.

¹H-NMR (400 MHz, CDCl₃, δ): 1,37; 1,49 (2d, 6H, 2 x -CH₃; J = 7,1 Hz); 1,47 (s, 9H, -OCMe₃); 2,34 (s, 3H, -N-CH₃); 3,51 (q, 1H, -O-CH-; J = 7,1 Hz); 3,74 (dd, 2H, Ph-CH₂-); 4,97 (q, 1H, -N-CH-; J = 7,1 Hz); 7,23 - 7,37 (m, 5H, arom.-H) ppm

EI-MS m/z (%): 321 (M⁺,1); 248 (2); 148 (Ph-CH₂-NMe-CHMe, 100); 91 (Ph-CH₂, 63)

### Beispiel (VI-1)

### N-Benzyl-N-methyl-L-isoleucyl-D-milchsäure

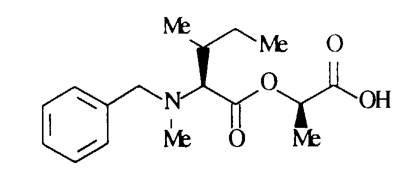

Die C-terminale Acidolyse erfolgt analog der Reaktionsvorschrift des Beispiels (II-3) unter Verwendung von:
25,0 g (68,8 mMol) N-Benzyl-N-methyl-L-isoleucyl-D-milchsäure-tert.-butylester,
500 ml Methylenchlorid.

Man erhält 21,0 g (99,3 % der Theorie) N-Benzyl-N-methyl-L-isoleucyl-D-milchsäure, die ohne weitere Reinigung weiter umgesetzt werden kann.

EI-MS m/z (%): 307 (M⁺,3); 250 (28); 190 (PhCH₂-NMe-CH-CHMeCH₂Me,100); 91 (PhCH₂, 85)

### Beispiel (VII-1)

### N-Methyl-L-isoleucyl-D-milchsäure-tert.-butylester

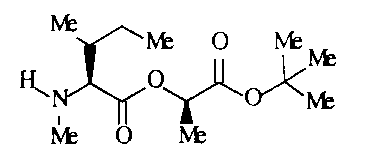

Die N-terminale Deblockierung erfolgt analog der Reaktionsvorschrift des Beispiels (II-5) unter Verwendung von:
25,0 g (68,8 mMol) N-Benzyl-N-methyl-L-isoleucyl-D-milchsäure-tert.-butylester,
500 ml Ethanol,
2,5 g Pd(OH)₂/Kohle [20 % Pd-Gehalt]

Man erhält 18,2 g (96,8 % der Theorie) N-Methyl-L-isoleucyl-D-milchsäure-tert.-butylester, der ohne weitere Reinigung weiter umgesetzt werden kann.

¹H-NMR (400 MHz, CDCl₃, δ): 1,47 (s, 9H, 3 x CH₃); 2,40 (s, 3H, -N-CH₃) ppm
Analog können die in den nachstehenden Tabellen 6 bis 8 aufgeführten Verbindungen der Formeln (V) - (VII) hergestellt werden.

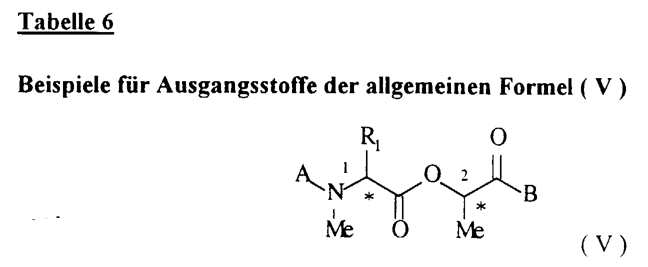

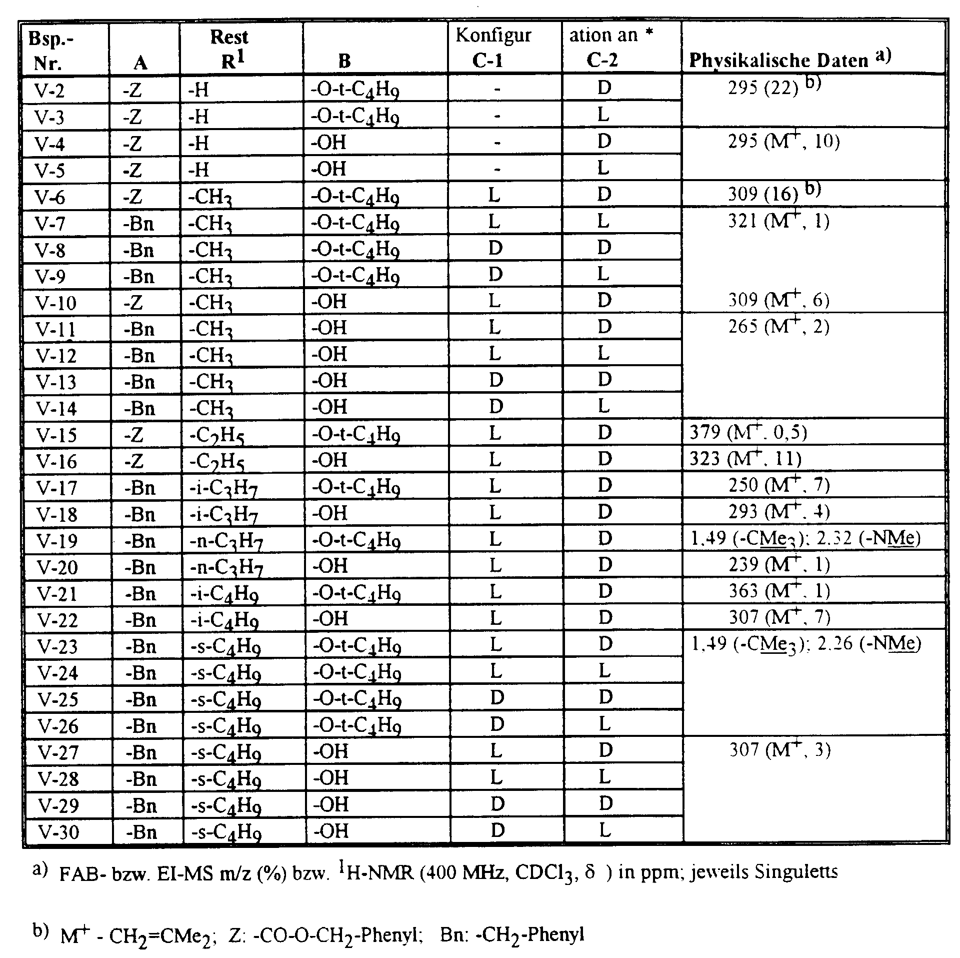

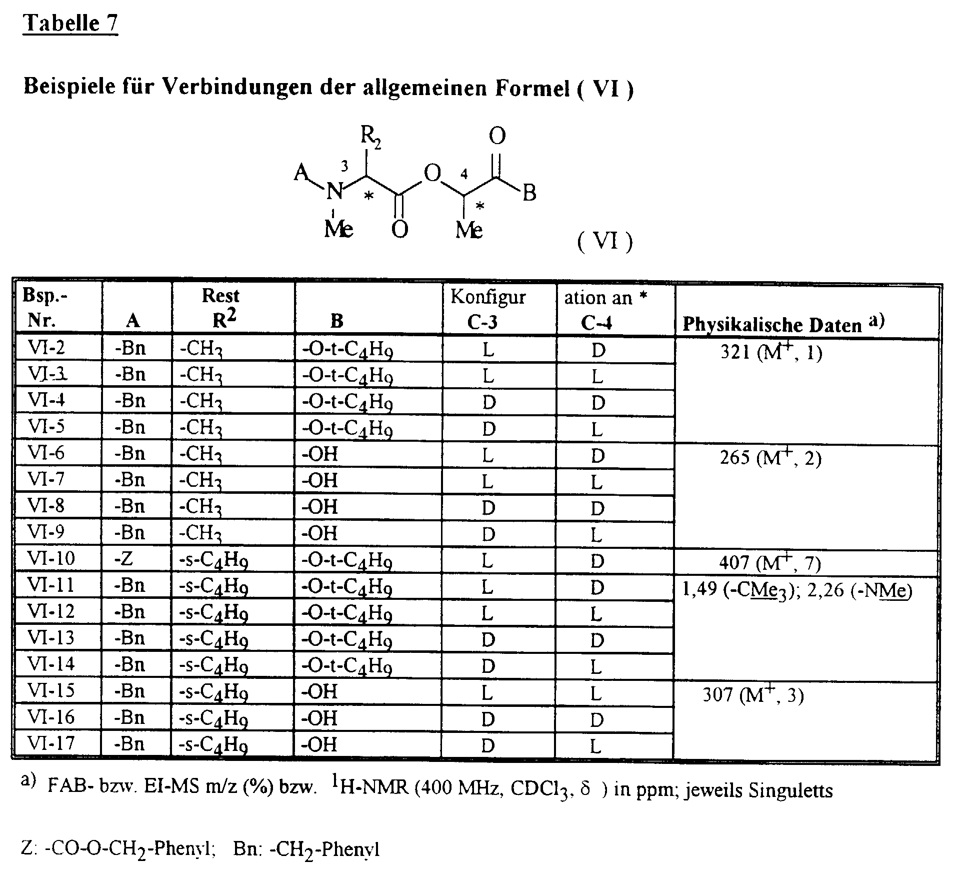

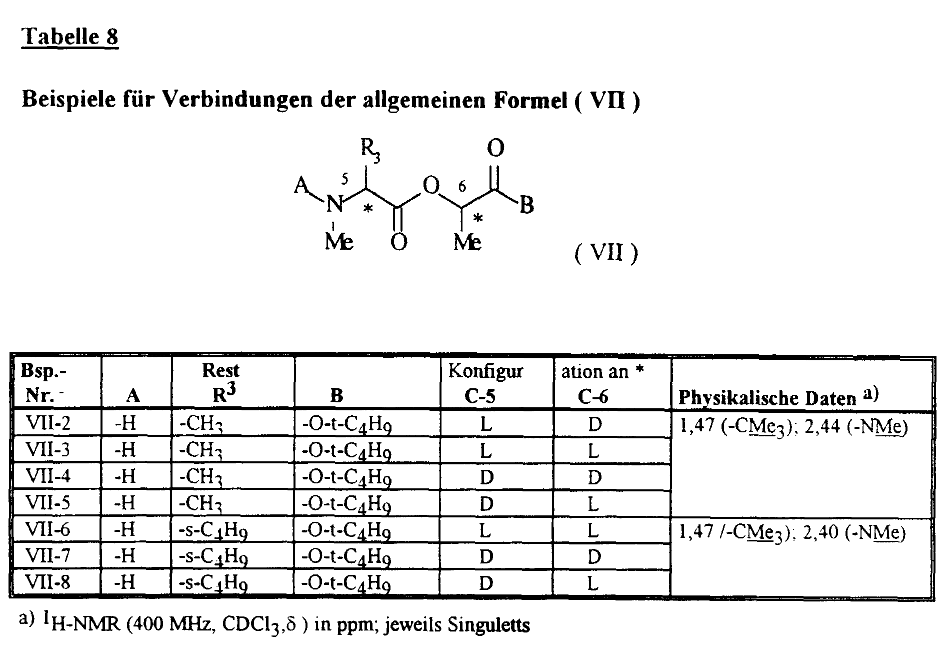

## Patentansprüche

1. Cyclische Depsipeptide mit 18 Ringatomen der allgemeinen Formel (I) 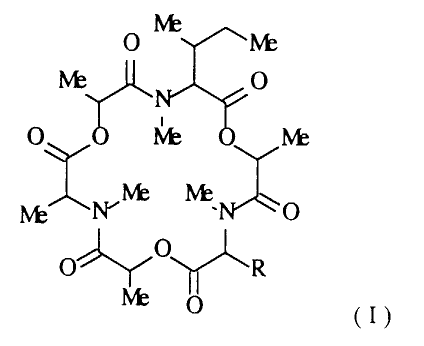 in welcher
R für Wasserstoff geradkettiges oder verzweigtes oder cyclisches Alkyl mit bis zu 8 Kohlenstoffatomen steht
sowie deren optische Isomere und Racemate.

2. Verfahren zur Herstellung der cyclischen Depsipeptide mit 18 Ringatomen der allgemeinen Formel (I) gemäß Anspruch 1 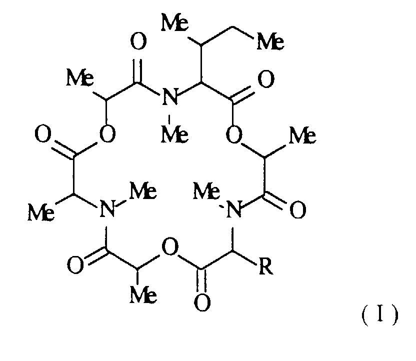 in welcher
R für Wasserstoff geradkettiges oder verzweigtes oder cyclisches Alkyl mit bis zu 8 Kohlenstoffatomen steht
sowie deren optische Isomere und Racemate,
dadurch gekennzeichnet, daß man
offenkettige Hexadepsipeptide der allgemeinen Formel (II) 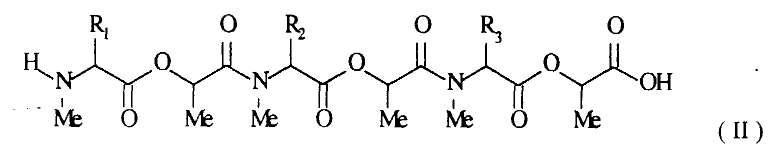 in welcher
einer der Reste R¹,R² und R³ für Wasserstoff, geradkettiges oder verzweigtes oder cyclisches Alkyl mit bis zu 8 Kohlenstoffatomen steht und die anderen beiden Reste für Methyl und sec.-Butyl stehen, in Gegenwart eines geeigneten Kupplungsreagenzes, in Gegenwart eines basischen Reaktionshilfsmittels und in Gegenwart eines Verdünnungsmittels cyclisiert.

3. Endoparasitizide Mittel, gekennzeichnet durch einen Gehalt an mindestens einem cyclischen Hexadepsipeptid der Formel (I) gemäß Anspruch 1.

4. Verfahren zur Herstellung von endoparasitiziden Mitteln, dadurch gekennzeichnet, daß man cyclische Hexadepsipeptide der Formel (I) gemäß Anspruch 1 mit Streckmitteln und/oder oberflächenaktiven Mitteln vermischt.

5. Verwendung von cyclischen Hexadepsipeptiden der Formel (I) gemäß Anspruch 1 zur Herstellung von endoparasitiziden Mitteln.

## Claims

1. Cyclic depsipeptides having 18 ring atoms, of the general formula (I) 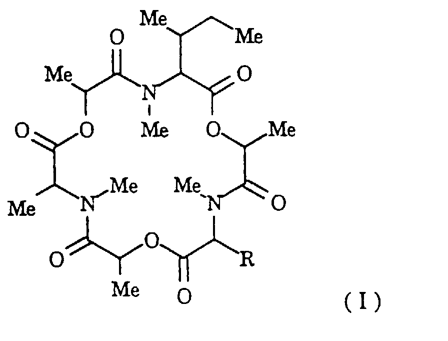 in which
R represents hydrogen, straight-chain or branched or cyclic alkyl having up to 8 carbon atoms
and their optical isomers and racemates.

2. Process for the preparation of the cyclic depsipeptides having 18 ring atoms, of the general formula (I) according to Claim 1 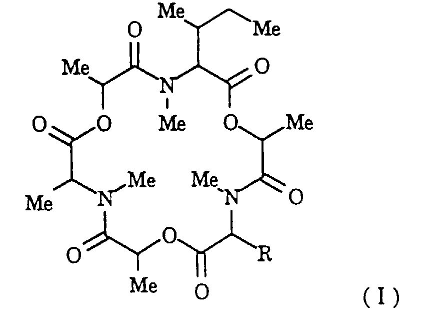 in which
R represents hydrogen, straight-chain or branched or cyclic alkyl having up to 8 carbon atoms
and their optical isomers and racemates,
characterized in that
open-chain hexadepsipeptides of the general formula (II) 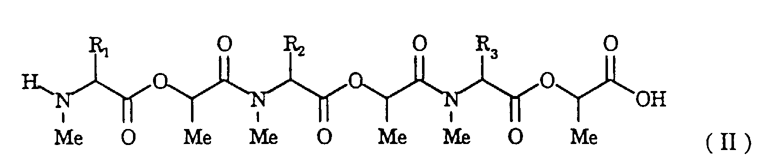 in which
one of the radicals R¹, R² and R³ represents hydrogen, straight-chain or branched or cyclic alkyl having up to 8 carbon atoms and the two other radicals represent methyl and sec-butyl, are subjected to a cyclization reaction in the presence of a suitable coupling reagent, in the presence of a basic reaction auxiliary and in the presence of a diluent.

3. Endoparasiticidal compositions, characterized in that they contain at least one cyclic hexadepsipeptide of the formula (I) according to Claim 1.

4. Process for the preparation of endoparasiticidal compositions, characterized in that cyclic hexadepsipeptides of the formula (I) according to Claim 1 are mixed with extenders and/or surfactants.

5. Use of cyclic hexadepsipeptides of the formula (I) according to Claim 1 for the preparation of endoparasiticidal compositions.

## Revendications

1. Depsipeptides cycliques à noyau de 18 atomes, de formule générale (I) 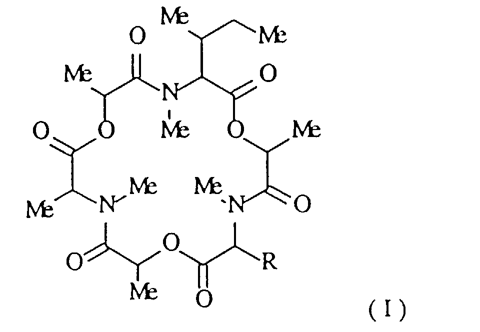 dans laquelle
R représente l'hydrogène, un groupe alkyle linéaire ou ramifié ou cyclique ayant jusqu'à 8 atomes de carbone
ainsi que leurs isomères optiques et leurs racémates.

2. Procédé de production des depsipeptides cycliques à noyau de 18 atomes de formule générale (I) suivant la revendication 1 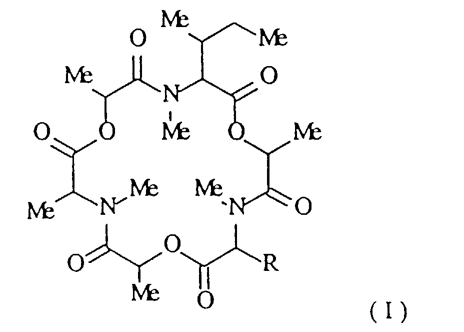 dans laquelle
R représente l'hydrogène, un groupe alkyle linéaire ou ramifié ou cyclique ayant jusqu'à 8 atomes de carbone
ainsi que de leurs isomères optiques et leurs racémates,
caractérisé en ce qu'on cyclise des hexadepsipeptides à chaîne ouverte de formule générale (II) 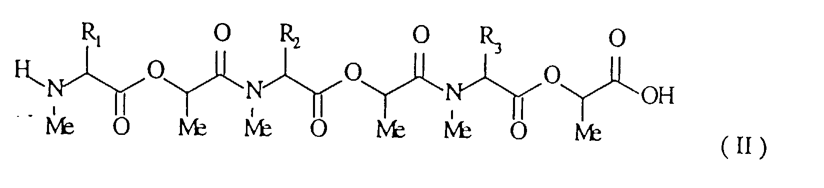 dans laquelle
l'un des restes R¹, R² et R³ représente l'hydrogène, un groupe alkyle linéaire ou ramifié ou cyclique ayant jusqu'à 8 atomes de carbone et les deux autres restes représentent les groupes méthyle et sec.-butyle, en présence d'un réactif de couplage approprié, en présence d'une substance auxiliaire basique de réaction et en présence d'un diluant.

3. Compositions endoparasiticides, caractérisées par une teneur en au moins un hexadepsipeptide cyclique de formule (I) suivant la revendication 1.

4. Procédé de préparation de compositions endoparasiticides, caractérisé en ce qu'on mélange des hexadepsipeptides cycliques de formule (I) suivant la revendication 1 avec des diluants et/ou des agents tensioactifs.

5. Utilisation d'hexadepsipeptides cycliques de formule (I) suivant la revendication 1 pour la préparation de compositions endoparasiticides.
